(19) Europäisches Patentamt
European Patent Office
Office européen des brevets

(11) EP 3 884 963 A1

(12) EUROPEAN PATENT APPLICATION

(43) Date of publication:
**29.09.2021 Bulletin 2021/39**

(21) Application number: **20382234.1**

(22) Date of filing: **26.03.2020**

(51) Int Cl.:
***A61K 47/69*** *(2017.01)* ***A61P 29/00*** *(2006.01)*
***A61K 9/51*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Consejo Superior de Investigaciones Científicas (CSIC)**
**28006 Madrid (ES)**

(72) Inventors:
- **ESPINOSA CANO, Eva**
  **28006 Madrid (ES)**
- **AGUILAR DE ARMAS, María Rosa**
  **28006 Madrid (ES)**
- **SAN ROMÁN DEL BARRIO, Julio**
  **28006 Madrid (ES)**
- **PORTILLA TUNDIDOR, Yadileiny**
  **28049 Madrid (ES)**
- **BARBER CASTAÑO, Domingo F.**
  **28049 Madrid (ES)**

(74) Representative: **Pons**
**Glorieta Rubén Darío 4**
**28010 Madrid (ES)**

(54) **NAPROXEN/DEXAMETHASONE-BASED NANOPARTICLES**

(57) The invention relates to novel self-assembled polymeric nanoparticles that comprises covalently-linked non-steroidal anti-inflammatory drugs (NSAIDs) and physically entrapped corticosteroids, and its use to reduce inflammatory responses.

EP 3 884 963 A1

SEQUENCE LISTING

<110> Consejo Superior de Investigaciones Científicas (CSIC)

<120> Naproxen/dexamethasone-based nanoparticles

<130> EP1641.1516

<160> 14

<170> PatentIn version 3.5

<210> 1
<211> 20
<212> DNA
<213> Artificial Sequence

<220>
<223> Forward primer Il23a

<400> 1
aataatgcta tggctgttgc 20

<210> 2
<211> 22
<212> DNA
<213> Artificial Sequence

<220>
<223> Reverse primer Il23a

<400> 2
cttagtagat tcatatgtcc cg 22

<210> 3
<211> 20
<212> DNA
<213> Artificial Sequence

<220>
<223> Forward primer Tnfa

<400> 3
ctatgtctca gcctcttctc 20

<210> 4
<211> 20
<212> DNA
<213> Artificial Sequence

<220>
<223> Reverse primer Tnfa

<400> 4
catttgggaa cttctcatcc 20

<210> 5
<211> 20
<212> DNA

<213> Artificial Sequence

<220>
<223> Forward primer Il12b

<400> 5
catcagggac atcatcaaac 20

<210> 6
<211> 21
<212> DNA
<213> Artificial Sequence

<220>
<223> Reverse primer Il12b

<400> 6
ctctgtctcc ttcatctttt c 21

<210> 7
<211> 20
<212> DNA
<213> Artificial Sequence

<220>
<223> Forward primer Vegfa

<400> 7
tagagtacat cttcaagccg 20

<210> 8
<211> 20
<212> DNA
<213> Artificial Sequence

<220>
<223> Reverse primer Vegfa

<400> 8
tctttctttg gtctgcattc 20

<210> 9
<211> 21
<212> DNA
<213> Artificial Sequence

<220>
<223> Forward primer Tgfb1

<400> 9
ggataccaac tattgcttca g 21

<210> 10
<211> 20
<212> DNA
<213> Artificial Sequence

<220>

```
<223>  Reverse primer Tgfb1

<400>  10
tgtccaggct ccaaatatag                                                    20


<210>  11
<211>  21
<212>  DNA
<213>  Artificial Sequence

<220>
<223>  Forward primer Il10

<400>  11
caggacttta agggttactt g                                                  21


<210>  12
<211>  20
<212>  DNA
<213>  Artificial Sequence

<220>
<223>  Reverse primer Il10

<400>  12
attttcacag ggagaaatc                                                     20


<210>  13
<211>  20
<212>  DNA
<213>  Artificial Sequence

<220>
<223>  Forward primer Actb

<400>  13
gatgtatgaa ggctttggtc                                                    20


<210>  14
<211>  20
<212>  DNA
<213>  Artificial Sequence

<220>
<223>  Reverse primer Actb

<400>  14
tgtgcacttt tattggtctc                                                    20
```

## Description

The invention relates to novel self-assembled polymeric nanoparticles that comprises covalently-linked non-steroidal anti-inflammatory drugs (NSAIDs) and physically entrapped corticosteroids (CS) such us dexamethasone (Dx), and its use to reduce inflammatory responses.

## BACKGROUND ART

Conventional medications such as NSAIDs and CS are first-line therapies for rheumatoid arthritis (RA) among other autoinmune-inflammatory diseases. They present rapid symptomatic effects compared with slower-acting disease-modifying antirheumatic drugs and biologic agents. CS have long been used for treatment of RA owing to their potent anti-inflammatory and immunosuppressive properties. They provide rapid control and improvement of inflammatory symptoms and, more importantly, they exhibit disease-modifying effects. NSAIDs are widely prescribed for their potent analgesic, antipyretic, and anti-inflammatory properties. However, they are not disease modifying when used alone, thus providing the basis for its frequent use in combination therapy for RA and other autoinmune-inflammatory diseases. Combination therapy has now become standard practice in treatment of RA.

Recent studies demonstrated the additive immunosuppressive and anti-inflammatory effect of dexamethasone (Dx) and naproxen (NAP) combined treatment with *in vitro* and *in vivo* studies on paw edema in female and male Lewis rats with collagen-induced arthritis (CIA).

CIA is a murine model of rheumatoid arthritis (RA), an autoimmune/inflammatory disorder, in which T cells are the primary dysfunctional cells. Interleukin-12 (IL12) and interleukin-23 (IL23) are secreted by macrophages and dendritic cells in response to antigen presentation to naive T cells playing a key role in the differentiation and maintenance of autoreactive T cells. These ILs are dimeric cytokines shearing IL12-p40 subunit which is encoded by IL12b gene. Actual biological treatments based on monoclonal antibodies blocking IL12-p40 subunit (e.g. ustekinumab) have demonstrated promising results and low side effects if compared with traditional tumor necrosis factor-alpha (TNF-$\alpha$) inhibitors.

Naproxen is a well-known non-steroidal anti-inflammatory drug (NSAID) with anti-inflammatory, antipyretic, and analgesic properties. It is associated to the lowest cardiovascular risk of all NSAIDs and its half-life is more than ten times higher than that of other NSAIDs. Naproxen derivatives have recently demonstrated capacity to inhibit IL12 production. However, naproxen is not a disease modifying drug when used alone, thus it is frequently combined with other drugs for the treatment of inflammatory processes.

Dexamethasone is one of the most frequently used therapeutic glucocorticoids. Owing to its immunosuppressive, and anti-inflammatory capacity, it has demonstrated great potential for the treatment of RA, Alzheimer or inflammatory Bowel disease. At low doses (i.e. below 100nM) dexamethasone treatment leads to polarization of *in vitro* cultured macrophages to M2 anti-inflammatory phenotype whereas at higher doses dexamethasone (approx. 1 $\mu$M) treatment leads to trans-repression of genes encoding for pro-inflammatory cytokines like IL12-p40 subunit and reduces vascular endothelial growth factor (VEGF) expression, a major contributor to macrophages-derived angiogenic activity.

Both drugs are first-line therapies for inflammation-related diseases because of their rapid symptomatic effects. However, their sustained use is limited by their high hydrophobicity, the rapid clearance of free drug from inflammation sites by the lymphatic system and the dose and time-dependent adverse effects. It has been well-established the enhanced therapeutic potential of nanomedicines over freely administered drugs. If they are properly designed, they preferentially accumulate in the pathological tissue providing a better spatial and temporal localization of the drug, and increasing its activity while reducing its adverse effects. Features like leaky vasculature or acidic pH characterize the inflamed microenvironment when compared with healthy tissue and they can be exploited to favor drug accumulation at inflamed areas. Matsumura et al. described for the first time that long-circulating macromolecules undergo passive accumulation in the inflamed tissue as they do in tumor sites through a mechanism similar to Enhanced Permeability and Retention (EPR) effect known as Enhanced Leaky Vasculature and Inflammatory cells Sequestration (ELVIS) effect. This name owes to the leaky vasculature that allows preferential accumulation at inflamed areas and the need of sequestration by inflammatory cells to avoid rapid clearance of the macromolecules or nanocarriers by lymphatic drainage which not present in tumors. Consequently, retention at inflamed tissue can be improved by tailoring nanoparticles (NPs) hydrodynamic properties (e.g. size and surface charge). Cheng et al. (Cheng, C. J.; Tietjen, G. T.; Saucier-Sawyer, J. K.; Saltzman, W. M., Nature reviews. Drug discovery 2015, 14, (4), 239-247) reported that NPs with diameter below 100 nm are easily cleared by lymphatic vessels and Awaad et al. (Awaad, A.; Nakamura, M.; Ishimura, K., Nanomedicine 2012, 8, (5), 627-636) concluded that 95-200 nm is the ideal size for increased cellular uptake. Moreover, NPs presenting positive surface charge showed higher cellular uptake when compared to neutral or anionic particles due to the ionic attraction between cationic charges and negatively charged phospholipids that composes the cell membrane.

The molecular basis of this synergistic immunosuppressive and anti-inflammatory capacity of dexamethasone and naproxen in collagen-induced arthritis in rats effect is barely understood, although downregulation of pro-inflammatory cytokines production has been involved. The low solubility of these drugs and their adverse effects hamper their efficacy

on the treatment of inflammatory processes being nanoparticulated systems promising candidates to overcome these drawbacks.

## SUMMARY OF THE INVENTION

New self-assembled bioactive NSAID (preferrentially naproxen (NAP) and ketoprofen (KT))-containing polymeric NPs loaded with dexamethasone were successfully prepared. Hydrodynamic properties and surface charge were optimized to improve retention and co-localization of both drugs at inflammation sites by the ELVIS effect. Dexamethasone was efficiently encapsulated and, *in vitro* biological studies demonstrated that both systems were non-cytotoxic and that dexamethasone encapsulation does not compromise the NO reduction capacity at any of the tested concentrations.

Finally, at the molecular level, it was observed the synergistic reduction of *IL12b* transcript levels when combining dexamethasone and NSAID. A clear dexamethasone dose-sparing capacity of NSAIDs was observed in terms of *IL12b* repression which makes NSAID/Dx combination highly interesting in the treatment of autoimmune-inflammatory diseases in which IL12 or IL23 are overexpressed and Th1 and Th17 cells play a key role in pathogenesis. Therefore, these drug delivery systems have potential to achieve better spatiotemporal localization of Dx/NSAID combination at inflamed areas and, by the synergistic reduction of IL12b transcript levels, they might improve the treatment of a wide range of autoimmune-inflammatory diseases.

Firstly, the methacrylic derivative of NSAID (preferentially naproxen (HNAP) or ketoprofen (HKT)) were successfully synthesized **(Scheme 1)** in mild conditions with yields above 90% and high purity after a simple purification procedure as confirmed by $^1$H-NMR spectroscopy **(Figure 1).** The linkage of the corresponding NSAID through the carboxylic group might improve safety profile as it has been reported as the main contributor to gastrointestinal adverse effects. The resultant ester linkage is prone to hydrolysis under acidic conditions encountered in inflamed tissues where the pH is one point lower than physiological *pH* (*i.e.* about 6.4) and in lumen of lysosomes whenever sequestration by inflammatory cells occurs. Altogether may provide a pH-accelerated release of NSAID at the inflamed area.

Next, the NSAID-containing copolymers (preferrentially poly(HNAP-co-VI) or poly(HKT-co-VI) for NAP or KT, respectively) were obtained via free radical polymerization reaction in solution. An amphiphilic gradient microstructure is obtained when copolymerizing low reactive hydrophilic vinylic monomers (preferably vinyl-imidazole (VI)) and highly reactive methacrylic ones (methacrylic derivate of NSAID, preferably HNAP or HKT) by free radical copolymerization. Moreover, if the hydrophobic-hydrophilic balance is the appropriate one this microstructure may self-assemble into NPs in aqueous media having a hydrophobic core mainly formed by covalently linked naproxen and a hydrophilic shell preferably formed by VI. As VI also presents protonable amine groups it may provide a positive surface charge to favor internalization by inflammatory cells. Accordingly, macromolecules with 0.5 feed molar content of HNAP or 0.4 feed molar content of HKT were prepared in order to have an appropriate hydrophobic-hydrophilic balance for copolymers self-assembly into NPs in aqueous media. The obtained copolymers are labeled according to their molar content in methacrylic derivative of NSAID (e.g. HNAP or HKT for naproxen and ketoprofen, respectively) obtained by $^1$H-NMR. As an example, the chemical reactions carried out for the synthesis of HNAP or HKT (a) and the copolymers (b) are illustrated in **Scheme 1 (Example 3).**

Hydrodynamic properties of the NSAID-based NPs (i.e. size, polydispersity and surface charge) were optimized as a function of pH, NPs final concentration ([NPs]F), and final volume (VF) to favor retention and co-localization of both drugs at inflammation sites through the ELVIS effect. Stability studies over time were also carried out. Dexamethasone was encapsulated and the encapsulation efficacy and loading capacity were determined by HPLC. *In vitro* biological studies using murine macrophages (RAW264.7) as model inflammatory cells were carried out to evaluate rapid uptake of NPs, cytotoxicity, anti-inflammatory capacity of the system and, the expression of genes related to autoimmune diseases.

Therefore, a first aspect of the present invention relates to novel self-assembled polymeric nanoparticles comprising hydrophobic monomeric NSAID-based units, wherein the nanoparticle is loaded with a corticosteroid.

In a preferred embodiment the NSAID is selected from salycilates (aspirin, diflunisal), carbo and heterocyclic acids (indomethacin, suldinac, tolmetin, etodolac), ketorolac, phenylacetic acids (diclofenac, fenclofenac, alcofenac), propinoic acids (naproxen, ketoprofen, ibuprofen, flurbiprofen, fenoprofen, fenbufen, oxaprozin, tiaprofenic acid), fenamic acids (mefenamics, meclofenamics, flufenamics, tolfenamics), and lumiracoxib; and more preferably the NSAID is naproxen or ketoprofen; even more preferably the NSAID is naproxen.

Therefore, in a preferred embodiment the hydrophobic monomeric NSAID-based unit is selected from from ketoprofen-based methacrylic monomer and naproxen-based methacrylic monomer; and more preferably is naproxen-based methacrylic monomer.

In a more preferred embodiment the polymeric nanoparticles also comprises hydrophilic monomers selected from N-vinylpyrrolidone, N,N-dimethylacrilamide, N-isopropylacrilamide, 2-hydroxyethyl metacrilate, 2-hydroxythyl acrilate, polyethylenglycol methacrylate, polyethylenglycol acrylate, 2-hydroxypropil methacrylate, N-ethylmorpholine methacrylate, N-ethylmorpholine acrylate, N-hydroxyethylpyrrolidone methacrylate, N-hydroxyethylpyrrolidone acrylate, 2-vinylpiridine and 1-vinyl imidazole; and more preferably the monomer is 1-vinyl imidazole.

In an even more preferably embodiment the polymeric nanoparticles are formed by self-assembly in aqueous media of copolymers which comprise monomeric units of naproxen-based methacrylic monomer and 1-vinyl imidazole, of high purity and substantially free of respective toxic monomeric contaminants.

In an even more preferably embodiment the polymeric nanoparticles are formed by self-assembly in aqueous media of copolymers which comprise monomeric units of ketoprofen-based methacrylic monomer and 1-vinyl imidazole, of high purity and substantially free of respective toxic monomeric contaminants.

In a preferred embodiment the corticosteroid is physically entrapped or encapsulated in the polymeric nanoparticle of the invention.

In another preferred embodiment the corticosteroid is selected from aclometasone, amcinonide, beclomethasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumethasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone furoate, paramethasone, prednicarbate, prednisone, prednisolone, prednylidene, remexolone, tixocortol, triamcinolone and ulobetasol, and combinations, pharmaceutically acceptable salts and esters thereof. In a more preferred embodiment, the corticosteroid is dexametasone.

A further embodiment of the present invention provides a method for preparing the polymeric nanoparticles as described before which comprises the following steps:

(a) synthesizing of hydrophobic monomeric NSAID-based units, preferably the methacrylic derivative of the NSAID-based unit;

(b) obtaining a polymer by free radical polymerization in solution of the the NSAID-based unit from step (a) or optionally a copolymer by free radical polymerization in solution of the NSAID-based unit of step (a) and monomers selected from N-vinylpyrrolidone, N,N-dimethylacrilamide, N-isopropylacrilamide, 2-hydroxyethyl metacrilate, 2-hydroxythyl acrilate, polyethylenglycol methacrylate, polyethylenglycol acrylate, 2-hydroxypropil methacrylate, N-ethylmorpholine methacrylate, N-ethylmorpholine acrylate, N-hydroxyethylpyrrolidone methacrylate, N-hydroxyethylpyrrolidone acrylate, 2-vinylpiridine and 1-vinyl imidazole; and more preferably the monomer is 1-vinyl imidazole.; and

(c) encapsulating a corticosteroid in polymer or optionally the copolymer obtained in step (b).

In a preferred embodiment the NSAID is selected from aspirin, diflunisal, indomethacin, suldinac, tolmetin, etodolac, ketorolac, diclofenac, fenclofenac, alcofenac, naproxen, ketoprofen, ibuprofen, flurbiprofen, fenoprofen, fenbufen, oxaprozin, tiaprofenic acid, mefenamics, meclofenamics, flufenamics, tolfenamics, and lumiracoxib; and more preferably the NSAID is naproxen or ketoprofen; even more preferably the NSAID is naproxen.

In another preferred embodiment, the monomer in step (b) is 1-vinyl imidazole.

In another preferred embodiment the polymeric nanoparticles of the invention are prepared by reacting a naproxen monomer (HNAP) and 1-vinyl imidazole in step (b).

In another preferred embodiment the polymeric nanoparticles of the invention are prepared by reacting a ketoprofen monomer (HKT) and 1-vinyl imidazole in step (b).

In another preferred embodiment the corticosteroid is selected from aclometasone, amcinonide, beclomethasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumethasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone furoate, paramethasone, prednicarbate, prednisone, prednisolone, prednylidene, remexolone, tixocortol, triamcinolone and ulobetasol, and combinations, pharmaceutically acceptable salts and esters thereof. In a more preferred embodiment, the corticosteroid is dexametasone.

A further embodiment of the present invention provides polymeric nanoparticles as described before for use as a medicament.

A further embodiment of the present invention provides polymeric nanoparticles as described before for use to reduce inflammatory responses and/or in the treatment of autoimmune-inflammatory diseases including between others Crohn's disease, ulcerative colitis, psoriatic arthritis, rheumatoid arthritis, ankylosing spondylitis, human multiple sclerosis, and psoriasis.

In the present invention the term "self-assembled nanoparticles" refers to nanoparticles based on amphiphilic polymers that maintains their organized structure and integrity by intermolecular interactions.

Unless otherwise defined, all technical and scientific terms used herein have the same meaning as commonly

understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

## DESCRIPTION OF THE DRAWINGS

**FIG. 1.** $^1$H-NMR spectrum of the product of the naproxen-based monomer synthesis.

**FIG. 2.** $^1$H-NMR spectrum of HNAP71 copolymer and the integrals used to compute copolymer molar composition.

**FIG. 3.** $^1$H-NMR spectrum of the product of the ketoprofen-based monomer synthesis.

**FIG. 4.** $^1$H-NMR spectrum of HKT48 copolymer and the integrals used to compute copolymer molar composition.

**FIG. 5.** Surface diagrams representing the variation of the instantaneous molar fraction of HNAP as a function of HNAP copolymer molar fraction and conversion.

**FIG. 6.** Surface diagrams representing the variation of the instantaneous molar fraction of HKT as a function of VI block mean length in the copolymer and conversion.

**FIG.7.** Scanning Electron Microscopy micrograph showing morphology of HNAP71-based NPs.

**FIG.8.** Size distribution (by intensity) and z-potential ($\xi$) of NPs based on HNAP71 measured a) at different pH values, b) at pH 4 and 4°C for different periods of time and; c) before freeze-drying (dashed line) and after freeze-drying and tip sonication for 10 minutes (solid line).

**FIG.9.** Scanning Electron Microscopy micrograph showing morphology of HKT48-based NPs.

**FIG. 10.** Size distribution (by intensity) and z-potential ($\xi$) of NPs based on HKT48 measured a) at different pH values, b) at day 0 and after 28 days stored at 4 °C and; c) before freeze-drying (dashed line) and after freeze-drying and tip sonication for 10 minutes.

**FIG. 11.** Mass of dexamethasone encapsulated measured by HPLC for each % Dx (w/w) with respect to the copolymer: HNAP71 (black) or HKT48 (grey). Results represent mean $\pm$ SD of two independent experiment, n = 2, $^*p \leq 0.05$. %Dx* in the x-axis refers to the statistical differences ($^*p \leq 0.05$) between Dx-HNAP71 and Dx-HKT48 systems.

**FIG.12.** Cell viability of RAW264.7 macrophages treated with different concentrations of unloaded NAP NPs (black), 8Dx-NAP NPs (white) or free Dexamethasone (red) over 24 h. The diagrams include the mean, the standard deviation (n = 8), and the ANOVA results ($^*p < 0.05$ statistically significant difference with cells treated with media (CP) and between the different systems: NAP NPs, NAP NPs-8Dx and free Dx).

**FIG. 13.** Cell viability of RAW264.7 macrophages treated with **a)** different concentrations of unloaded KT NPs (black), 14Dx-KT NPs (white) or free Dx (red); **b)** unloaded NAP NPs (black) and unloaded KT NPs (white); and **c)** 8Dx-NAP NPs (black) and 14Dx-KT NPs (white) over 24 h. The diagrams include the mean, the standard deviation (n = 8), and the ANOVA results ($^*p < 0.05$ statistically significant difference with cells treated with media (CP) and between the different systems).

**FIG.14.** Schematic presentation of the polarization of murine macrophages in response to lipopolysaccharide (LPS) or dexamethasone (Dx) activation and the relation of $M_1$- and $M_2$-marker genes under study in this work.

**FIG. 15.** NO release by RAW264.7 macrophages after 24 h with no treatment (LPS-), treatment with LPS alone (LPS+), and treatment with LPS and different concentrations of unloaded NAP NPs (black), 8Dx-NAP NPs (white) or free Dexamethasone (red) suspension. The diagrams include the mean, the standard deviation (n = 8), and the ANOVA results ($^{***}p < 0.05$ and $^\#p < 0.01$ statistically significant difference with LPS+ cells or between NAP NPs, 8Dx-NAP NPs and free dexamethasone).

**FIG. 16.** Quantitative real-time PCR data. Gene transcript levels of a) M1 markers or b) M2 markers in non-inflamed samples (no LPS) treated with free dexamethasone (free Dx, 2.55 $\mu$M), Dx-loaded naproxen-bearing NPs (8Dx-NAP NPs, 2.55$\mu$M Dx and 0.045 mg/mL NPs) and unloaded naproxen-bearing NPs (NAP NPs, 0.045 mg/mL) for 1 day or 7 days. Results are expressed relative to the corresponding level of expression of each transcript in the untreated sample. Data are presented as mean fold changes in gene transcript levels in two independent samples per system.

**FIG. 17.** Quantitative real-time PCR data. Diagrams including the mean, the standard deviation (n = 2), and the ANOVA results (# - comparison with LPS+ control, $^\#p < 0.05$ and $^{\#\#\#}p < 0.001$; * - comparison with untreated LPS- control, $^*p < 0.05$, $^{**}p < 0.01$ and $^{***}p < 0.001$) of the gene transcript levels of M1 markers in inflamed samples (500 ng/mL of LPS) treated with just LPS (LPS+) or LPS and free dexamethasone (free Dx, 2.55 $\mu$M), Dx-loaded naproxen-bearing NPs (8Dx-NAP NPs, 2.55 $\mu$M Dx and 0.045 $mgmL^{-1}$ NPs) and unloaded naproxen-bearing NPs (NAP NPs, 0.045 $mgmL^{-1}$) for 1 day or 7 days. Results are expressed relative to the corresponding level of expression of each

transcript in the untreated sample (LPS-). Data are presented as mean fold changes in gene transcript levels in two independent samples per system.

**FIG. 18.** NO release by RAW264.7 macrophages after 24 h (black) and 48 h (white) with no treatment (LPS-), treatment with LPS alone (LPS+), and treatment with LPS and different concentrations of unloaded NAP/KT NPs (upper row) or 8Dx-NAP NPs/14Dx-KT NPs (lower row). The diagrams include the mean, the standard deviation (n = 8), and the ANOVA results (*$p < 0.05$, ***$p < 10^{-3}$, and #$p < 10^{-5}$ statistically significant difference with LPS+ cells or between 24 h and 48 h time points).

**FIG. 19.** NO release by RAW264.7 macrophages after 48 h with no treatment (LPS-), treatment with LPS alone (LPS+), and treatment with LPS and different concentrations of unloaded NAP NPs or KT NPs (black), 8Dx-NAP NPs or 14Dx-KT NPs (white) or free Dx (red). The diagrams include the mean, the standard deviation (n = 8), and the ANOVA results (*$p < 0.05$, ***$p < 10^{-3}$, and #$p < 10^{-5}$ statistically significant difference with LPS+ cells or between systems).

**FIG. 20.** Quantitative real-time PCR data. Diagrams including the mean, the standard deviation (n = 2), and the ANOVA results (# - comparison with LPS+ control, #$p < 0.05$ and ###$p < 0.001$; * - comparison with untreated LPS- control, *$p < 0.05$, **$p < 0.01$ and ***$p < 0.001$) of the gene transcript levels of M1 markers in inflamed samples (500 ng/mL of LPS) treated with just LPS (LPS+) or LPS and free dexamethasone (free Dx, 5.1 μM), Dx-loaded naproxen-bearing NPs (14Dx-KT NPs, 5.1 μM Dx and 0.045 $mgmL^{-1}$ NPs) and unloaded naproxen-bearing NPs (KT NPs, 0.045 $mgmL^{-1}$) for 1 day or 7 days. Results are expressed relative to the corresponding level of expression of each transcript in the untreated sample (LPS-). Data are presented as mean fold changes in gene transcript levels in two independent samples per system.

## EXAMPLES

### Materials and Methods

*Materials*

Dichloromethane (DCM, Aldrich), (+)-(S)-2-(6-methoxynaphthalen-2-yl) propanoic acid (naproxen, NAP, TCI), ketoprofen (KT, TCI), 4-dimethylaminopyridine (DMAP, Aldrich), dicyclohexylcarbodiimide (DCC, Fluka), hydroxyethyl methacrylate (HEMA, Aldrich), sodium bicarbonate ($NaHCO_3$, Panreac), sodium chloride (NaCl, Panreac) and magnesium sulphate ($MgSO_4$, Qemical) were used without further purification for the synthesis and purification of the NSAID-based methacrylic monomer. Dimethylsulfoxide (DMSO, Scharlau) was used without further purification, 1-vinylimidazole (VI, Aldrich) was distilled and 2,2'-azobisisobutyronitrile (AIBN, Merck) was recrystallized from ethanol for the preparation of amphiphilic copolymers. Deuteraded DMSO ($DMSOd_6$, Merck), deuterated chloroform ($CDCl_3$, Sigma-Aldrich) and chromatographic grade dimethylformamide (DMF, Scharlab) were used in the NMR and SEC experiments. Additionally, ethanol (EtOH, Scharlau) and, acetone (DMK, Scharlau) were used without further purification. DEXA (≥98% pure; CAS Number: 50-02-2) and lipopolysaccharide (LPS) from Escherichia coli O111:B4 (CAS Number: 297-473-0) were purchased from Sigma-Aldrich (Sigma-Aldrich; St. Louis, USA).

*Synthesis and characterization of methacrylic derivative of NSAID*

The synthesis of the methacrylic derivative of naproxen (HNAP) or ketoprofen (HKT) were carried out through a carbodiimide mediated Steglich esterification reaction. Briefly, NSAID (1 equiv) was dissolved in dichloromethane (DCM) and subsequently, purified hydroxyethyl methacrylate (HEMA, 1 equiv) and 4-dimethylaminopyridine (DMAP, 0.1 equiv) were also added to the reaction mixture. Dicyclohexylcarbodiimide (DCC, 1 equiv) was then added dropwise under continuous stirring and $N_2$ (g) atmosphere. The resultant solution was stirred overnight at room temperature. The reaction product was filtered to eliminate N,N'-diciclohexylurea (DCU) formed salt and the organic phase was washed with water and a saturated solution of sodium bicarbonate ($NaHCO_3$). Formerly, the resultant solution was dried with a saturated NaCl solution and anhydrous magnesium sulphate ($MgSO_4$) and finally, DCM was evaporated under reduced pressure. The structure and purity of the product was determined by proton nuclear magnetic resonance ($^1H$-NMR) performed in a Varian Mercury 400 MHz equipment using deuterated chloroform ($CDCl_3$) as a solvent at 25 °C.

*Synthesis of amphiphilic pseudo-gradient copolymers of naproxen-based methacrylic monomer and 1-vinyl imidazole, poly(HNAP-co-VI).*

The copolymer based on methacrylic derivative of naproxen and 1-vinylimidazole (VI) was prepared via free radical polymerization, a feed molar in HNAP ($F_{HNAP}$) of 0.5, and an initial monomers concentration ([M]) of 0.5 M were used. As a summary, both VI and HNAP were dissolved in dimethylsulfoxide (DMSO, Scharlau) and the reaction mixtures

were deoxygenated with $N_2$ (g) for 10 minutes. Then, 2,2'-azobisisobutyronitrile (AIBN, $1.5 \times 10^{-2}$ M) was added and the reaction was kept at 60°C for 12 hours. Copolymerization mixtures were dialyzed (3.5K molecular weight cut-off) against distilled water for 72 hours. The resultant copolymers were isolated by freeze-drying as a white powder.

*Synthesis of amphiphilic pseudo-gradient copolymers of ketoprofen-based methacrylic monomer and 1-vinyl imidazole, poly(HKT-co-VI).*

The copolymer based on methacrylic derivative of ketoprofen and 1-vinylimidazole (VI) was prepared via free radical polymerization, a feed molar in HKT ($F_{HKT}$) of 0.4, and an initial monomers concentration ([M]) of 0.5 M were used. As a summary, both VI and HKT were dissolved in dimethylsulfoxide (DMSO, Scharlau) and the reaction mixtures were deoxygenated with N2 (g) for 10 minutes. Then, 2,2'-azobisisobutyronitrile (AIBN, $1.5 \times$ 10-2 M) was added and the reaction was kept at 60°C for 12 hours. Copolymerization mixtures were dialyzed (3.5K molecular weight cut-off) against distilled water for 72 hours. The resultant copolymers were isolated by freeze-drying as a white powder.

*Characterization of copolymers*

*Proton Nuclear Magnetic Resonance ($^1$H-NMR) of polv(HNAP-co-VI).*

1H-NMR of the obtained copolymers were performed in a Varian Mercury apparatus operating at 400 MHz. Spectra were recorded by dissolving the samples in $DMSO_{d6}$ at 25 °C. Copolymers composition was calculated using MestreNova 9.0 from the $^1$H-NMR integral between 7.92-6.47 ppm corresponding to the aromatic protons of both monomers and the integral between 2.28-0.1 ppm which corresponds to protons of the methyl groups i and m of HNAP and protons l and n from the main hydrophobic carbon chain of HNAP and VI, respectively **(figure 2)**

*Proton Nuclear Magnetic Resonance ($^1$H-NMR) of poly(HKT-co-VI).*

$^1$H-NMR was performed in a Varian Mercury equipment operating at 400 MHz. Spectra was recorded by dissolving samples in deuteraded DMSO ($DMSO_{d6}$, Merck) at 25 °C. Copolymer composition was calculated using MestreNova 9.0 from the $^1$H-NMR integral between 7.92-6.47 ppm corresponding to the aromatic protons of both monomers and the integral between 2.28-0 ppm which corresponds to protons of the methyl groups k and o of HKT and protons 1 and n from the main hydrophobic carbon chain of VI and HKT, respectively **(figure 4).**

*Size Exclusion Chromatography (SEC).*

HKT48 and HNAP71 copolymers apparent average molecular weight (Mn and Mw) and polydispersity index (Đ) were determined by SEC, using a Perkin-Elmer Isocratic LC pump 250 coupled to a refraction index detector (Series 200). Two Resipore columns (250 mm x 4.6 mm, Varian) were used as solid phase, and degassed chromatographic grade dimethylformamide (DMF, Scharlab, 0.7 mL/min) with LiBr (0.1 % w/v) was used as eluent and temperature was fixed at 70 °C. Monodisperse PMMA standards (Scharlab) with molecular weights between 10,300 and 1,400,000 Da were used to obtain the calibration curve. Data was analysed using the Perkin-Elmer LC solution program.

*Differential Scanning Calorimetry (DSC).*

Glass transition temperature (Tg) was determined by differential Scanning Calorimetry (DSC) with a Perkin Elmer DSC8500 interfaced to a Pyris thermal analysis data system. Dried samples (3-5 mg) were placed in aluminium pans and heated from -20 to 120 °C at a constant speed of 20 °C/min. Tg was taken as the midpoint of the heat capacity transition.

*Determination of reactivity ratios of poly(HNAP-co-VI)*

Reactivity ratios of the comonomers were determined by "in situ" $^1$H-NMR monitorization of the copolymerization reaction according to a previously described protocol. Briefly, copolymerization reactions ($F_{HNAP}$ = 0.20, 0.40 and 0.60; [M] = 0.25 M and [AIBN] = 0.015 M) were carried out inside a NMR tube in the same experimental conditions as described above but using $DMSO_{d6}$ as a solvent. A solution of DMF (10 mg/mL) in $DMSO_{d6}$ in a thin wall capillary tube introduced in the NMR tube was used as reference. Temperature was maintained at 60 °C using the apparatus (Varian Mercury 400 MHz) heat control system. Signals were integrated using MestreNova 9.0 software and reactivity ratios were determined using the methodology described by Aguilar, M.R. et al. in 2002.

*Determination of reactivity ratios of poly(HKT-co-VI)*

Monomers reactivity ratios were calculated through "in situ" $^1$H-NMR monitorization of the copolymerization reaction following the second methodology described by Aguilar, M.R. et al. in 2002. Briefly, copolymerization reactions ($F_{HKT}$ = 20, 40 and, 60; [M] = 0.25 M and [AIBN] = 0.015 M) were carried out inside the NMR equipment (Varian Mercury 400 MHz) using DMSOd6 as a solvent at 60 °C, 90° pulse and acquiring a new spectrum each 120 seconds. A solution of DMF (10 mg/mL) in $DMSO_{d6}$ in a thin wall capillary tube introduced in the NMR tube was used as reference.

*Preparation of self-assembled HNAP71-based nanoparticles*

Self-assembled NPs were prepared via nanoprecipitation method. Briefly, copolymer,HNAP71, was dissolved in a 8:2, acetone:ethanol solvents mixture at a concentration of 10 mg/mL and then added dropwise over a continuously stirred aqueous buffer solution at pH 4 (0.1 M Acetic Acid and 100 mM NaCl), a pH below the reported $pK_b$ of VI (i.e. 5.5-6.1). The resultant suspension was stirred overnight to evaporate remaining organic solvent. Suspensions were stored in suspension at 4 °C.

*Preparation of self-assembled HKT48-based nanoparticles*

Self-assembled NPs were prepared via nanoprecipitation method. Briefly, copolymer, HKT48, was dissolved in a 8:2, acetone:ethanol solvents mixture at a concentration of 10 mg/mL and then added dropwise over a continuously stirred aqueous buffer solution at pH 4 (0.1 M Acetic Acid and 100 mM NaCl), a pH below the reported $pK_b$ of VI (i.e. 5.5-6.1). The resultant suspension was stirred overnight to evaporate remaining organic solvent. Suspensions were stored in suspension at 4 °C.

*Characterization of the NPs*

*Size distribution and surface charge*

The particle size distribution of the NP suspension was determined by dynamic light scattering (DLS) using a Malvern Nanosizer NanoZS Instrument equipped with a 4 mW He-Ne laser ($\lambda$ = 633 nm) at a scattering angle of 173°. Measurements of NP dispersions were performed in square polystyrene cuvettes (SARSTEDT) and the temperature was kept constant at 25 °C. The autocorrelation function was converted in an intensity particle size distribution with ZetaSizer Software 7.10 version, to get the mean hydrodynamic diameter ($D_h$) and the particle dispersion index (PDI) between 0 (monodisperse particles) and 1 (polydisperse particles) based on the Stokes-Einstein equation, assuming the particles to be spherical. For each sample, the statistical average and standard deviation of data were calculated from 3 measurements of 11 runs each one. The zeta potential ($\xi$) of NP dispersions was determined by laser Doppler electrophoresis (LDE) using a Malvern Nanosizer NanoZS Instrument. The $\xi$ statistical average and standard deviations were calculated from 3 measurements of 20 runs each one.

*Scanning electron microscopy NPs morphology characterization*

To determine NPs shape, a SEM analysis of HNAP71 and HKT48 unloaded NPs was performed with a Hitachi SU8000 TED, cold-emission FE-SEM microscope working with an accelerating voltage 1 kV-D. Sample preparation consisted in the deposition of one drop of the corresponding NPs suspension (0.02 mg/mL) over a small glass disk (12 mm diameter) and evaporation at room temperature. Gold palladium alloy (80:20) was used to coat the samples prior to examination by SEM.

*Optimization of final NP concentration ($[NPs]_F$) and final volume ($V_F$)*

Different final concentrations of NPs in aqueous suspension ($[NPs]_F$ = 1 mg/mL and 5 mg/mL) and three different final volumes of the aqueous phase ($V_F$ = 10 mL, 20 mL and 30 mL) were tested. Hydrodynamic properties were measured to determine the largest volume and final concentration that can be prepared keeping hydrodynamic properties within the appropriate ranges for cell uptake by inflammatory cells.

*Optimization of storage conditions*

A pH study was performed to determine the aggregation pH of the systems and set the appropriate storage pH. NPs were prepared at pH = 4.0 and hydrodynamic properties were measured. pH was increased by 0.5 via the

addition of 1*M* NaOH aqueous solution and size distribution and ξ were measured. The procedure was repeated until the aggregation pH was reached. After that, the pH was set to 4.0 again to check reversibility of the aggregation phenomenon. Samples size distribution and ξ were measured every week up to one month to demonstrate NPs stability when stored in suspension at 4 °C and at the most suitable storage pH. NPs were freeze-dried after preparation and dispersed in the same volume of 0.1 M acetic acid buffer solution. The suspension was then subjected to manual shaking and ultrasound bath/tip sonication (30 % amplitude) for 10 minutes. After sonication, size distribution of NPs was measured to check redispersibility.

*Dexamethasone encapsulation*

Dexamethasone (Dx, Aldrich, ≥98% pure; CAS Number: 50-02-2)-loaded NSAID-based NPs were also prepared by nanoprecipitation method. Dx (5, 10, 15, 20 or, 25% w/w with respect to the copolymer) and the corresponding copolymer (10 mg/mL) were dissolved in a mixture acetone:ethanol (8:2, v/v) and added dropwise to the previously mentioned aqueous buffer solution at pH 4 under magnetic stirring. The final polymer concentration was 3.0 mg/mL. The solution was dialyzed against 0.1 M acetic acid, 0.1 M NaCl aqueous solution at pH 4 for 72h in order to eliminate the organic solvent and the small concentration of soluble non-encapsulated Dx. The resultant NPs were filtered through 1 μm Nylon filters to remove remaining insoluble Dx.

*Encapsulation efficiency (%EE) and Loading Capacity (%LC)*

Dx-loaded NSAID-based NPs were freeze dried in order to eliminate aqueous phase. The obtained white amorphous powder was dissolved in 2 ml of a mixture acetone:ethanol (8:2, v/v) to disassembly the nanoparticulated structure and release the encapsulated drug; and the organic solvent is let to evaporate overnight. After that, the copolymer-Dx mixture was dissolved in a mixture acetonitrile:water (8:2, v/v). Samples were centrifuged at 10.000 rpm for 5 minutes at RT and supernatant was analyzed by HPLC (Dx, λabs = 239 nm). Encapsulation efficiency (%EE) was computed using Equation 1 and the loading capacity (%LC) using Equation 2.

$$\text{Encapsulation Efficiency (\%)} = \frac{[\text{c6}]_{\text{measured}}}{[\text{c6}]_{\text{initial}}} \times 100 \quad \textbf{(1)}$$

$$Loading\ capacity\ (\%) = \frac{mass\ of\ c6\ measured}{mass\ of\ NPs} \times\ 100\ \textbf{(2)}$$

*Cell culture*

The RAW264.7 (murine macrophages) cell line was purchased from Sigma-Aldrich. RAW264.7 cells are an *in vitro* model useful to test the potential anti-inflammatory activity of new drugs [22]. Cells were maintained over permissive conditions in high-glucose Dulbecco's modified Eagle's medium (DMEM; Sigma, Saint Louis, MO, USA) supplemented with 10% fetal bovine serum (FBS; Gibco, BRL), 2% L-Glutamine (Sigma, Saint Louis, MO, USA) and Penicillin-G (Sigma, Saint Louis, MO, USA) at 37 °C in a humidified incubator with 10% $CO_2$.

*In vitro viability assay of NPs*

2 x $10^5$ live cells/mL (100 μL per well) were seeded in a 96 well-plate under permissive conditions. After 24 h, cells were exposed to different concentrations of free Dx and Dx-loaded or unloaded NSAID-based NPs suspension (0.25, 0.125, 0.09, 0.045, 0.023 and, 0.011 mg/ml) and cell viability was determined after 24 h of incubation with NPs. AlamarBlue (Invitrogen) was used to determine cell viability. Absorbance at 570 nm was measured by a Multi-Detection Microplate Reader Synergy HT (BioTek Instruments; Vermont, USA). The treatments were done in replicates (n = 8). Results of the experiments were expressed as percentage of relative cell viability (% respect to the control).

*Test anti-inflammatory activity of NPs*

*Nitric Oxide (NO)* assay

2 x $10^5$ live cellsmL$^{-1}$ (100 μL per well) were seeded in a 96 well-plate under permissive conditions. After 24 h cells were treated with LPS (5 μgmL$^{-1}$) and with different $[NPS]_F$ of unloaded NAP or KT NPs, 8Dx-NAP NPs-8Dx or

14Dx-KT NPs or free Dx at the maximum concentration that is encapsulated on each sample. 24 h after free Dx or NPs addition, LPS-induced NO release was evaluated by Griess reagent modified kit (Sigma-Aldrich) following the manufacturer protocol. The treatments were done in replicates (n = 8). Results of the experiments were expressed as percentage of relative NO released (% respect to the control, cell activated with LPS (LPS+)) normalized by the number of cells obtained by Alamar Blue assay and considering NO level of cells activated with LPS 100%.

*RNA extraction, reverse transcription, real-time quantitative PCR (RT-qPCR)*

The transcription of $M_1$ (*IL12b, IL23a,* and *TNF-$\alpha$*), $M_2$ (*TGF-$\beta$,* and *IL 10*) and pro-angiogenic (*VEGF*) related genes were investigated by quantitative RT-PCR. RAW264.7 cells were incubated 24h with or without LPS (500 ng/mL) and either non-treated (CP) or treated with unloaded NAP NPs or KT NPs (0.045 mg/mL), 8Dx-NAP-NPs (2.55 $\mu$M Dx and 0.045 mg/mL NPs), 14Dx-KT NPs (5.1 $\mu$M Dx and 0.045 mg/mL NPs) or free Dx (2.55 $\mu$M (for NAP) or 5.1 $\mu$M (for KT)). After 24h, macrophages were washed with medium to eliminate non-internalized NPs or free Dx from the culture media and incubated in culture medium up to seven days. At day 1 and day 7, total RNA was extracted from cells using the PureLink RNA Mini Kit (Applied Biosystems) following manufacturer's instructions[62]. RNA concentration was determined by absorbance measurements at 260 and 280 nm in a NanoDrop 1000 spectrophotometer (Thermo Scientific) and 40 ng RNA/sample were converted into cDNA using a MultiScribe reverse transcription-based reaction kit (Applied Biosystems) in the presence of a RNAse inhibitor (N8080119, Applied Biosystems) in a MyCycler thermocycler (Bio-Rad; with the following temperature profile: 25°C-10 min, 37°C-2h, 85°C-5 min, 4°C $\infty$). Specific primers were used for quantitative PCR. The list of specific primers used (all from Sigma) is provided in **Table 1.**

**Table 1.** RT-qPCR primer list

| Gene Symbol | Official Full Name | Species | Forward (5'-3') | Reverse (5'-3') |
|---|---|---|---|---|
| ***Il23a*** | Interleukin-23 subunit alpha | mouse | AATAATGCTAT GGCTGTTGC (SEQ ID NO: 1) | CTTAGTAGATT CATATGTCCCG (SEQ ID NO: 2) |
| ***Tnfa*** | Tumor necrosis factor | mouse | CTATGTCTCAG CCTCTTCTC (SEQ ID NO: 3) | CATTTGGGAAC TTCTCATCC (SEQ ID NO: 4) |
| ***Il12b*** | Interleukin-12 subunit beta | mouse | CATCAGGGACA TCATCAAAC (SEQ ID NO: 5) | CTCTGTCTCCT TCATCTTTTC (SEQ ID NO: 6) |
| ***Vegfa*** | Vascular endothelial growth factor A | mouse | TAGAGTACATC TTCAAGCCG (SEQ ID NO: 7) | TCTTTCTTTGGT CTGCATTC (SEQ ID NO: 8) |
| ***Tgfb1*** | Transforming growth factor beta-1 proprotein | mouse | GGATACCAACT ATTGCTTCAG (SEQ ID NO: 9) | TGTCCAGGCTC CAAATATAG (SEQ ID NO: 10) |
| ***Il10*** | Interleukin-10 | mouse | CAGGACTTTAA GGGTTACTTG (SEQ ID NO: 11) | ATTTTCACAGG GGAGAAATC (SEQ ID NO: 12) |

(continued)

| Gene Symbol | Official Full Name | Species | Forward (5'-3') | Reverse (5'-3') |
|---|---|---|---|---|
| ***Actb*** | Actin, beta | mouse | GATGTATGAAG GCTTTGGTC (SEQ ID NO: 13) | TGTGCACTTTT ATTGGTCTC (SEQ ID NO: 14) |

The reaction was performed using the Power SYBR Green PCR Master Mix (Applied Biosystems), in an ABI PRISM 7900HT Real-Time PCR System (Applied Biosystems; with the following temperature profile: 95°C-15 s, 60°C-60 s, 40 cycles). Melting curves were generated in order to verify the specificity of the amplification (15 s, from 60°C to 95°C). RT-qPCR expression data were analyzed according to the $2^{-\Delta\Delta Ct}$ method (Livak et al., 2001) or as $2^{-\Delta Ct}$ normalized to β-actin. For global analysis, x fold change values were translated to a heat map representation with MeV software (Institute for Genomic Research, Rockville MD). All quantifications were performed in triplicate.

**Example 1. Methacrylic derivative of naproxen monomer (HNAP)**

The synthesis of the HNAP was carried out through a carbodiimide mediated Steglich esterification reaction **(Scheme 1)** as described in Suárez, P. et al. (Macromol. Biosci. 2013, 13, (9)) for the methacrylic derivative of ibuprofen. Briefly, naproxen (1 equiv) was dissolved in dichloromethane and subsequently, purified hydroxyethyl methacrylate (HEMA, 1 equiv) and 4-dimethylaminopyridine (DMAP, 0.1 equiv) were also added to the reaction mixture. DCC (1 equiv) was then added dropwise under continuous stirring and $N_2$ (g) atmosphere. Afterwards, the resultant solution was stirred for 24h at room temperature. The reaction product was filtered to eliminate *N,N'*-diciclohexylurea (DCU) formed salt and the organic phase was washed with water and a saturated solution of $NaHCO_3$. Formerly, the resultant solution was dried with a saturated NaCl solution and anhydrous $MgSO_4$ and finally, DCM was evaporated under reduced pressure. The yield of the reaction was 90 %. The chemical structure and purity of HNAP was elucidated by $^1$H-NMR spectroscopy.
***HNAP:*** *$^1$H NMR (400 MHz, $CDCl_3$) $\delta_H$ 7.66 - 7.51 (m, 3H), 7.32 (dd, J = 8.5, 1.9 Hz, 1H), 7.12 - 6.98 (m, 2H), 5.87 (dq, J = 1.9, 1.0 Hz, 1H), 5.37 (p, J = 1.6 Hz, 1H), 4.32 - 4.13 (m, 4H), 3.84 (s, 4H), 1.74 (dd, J = 1.6, 1.0 Hz, 3H), 1.51 (d, J = 7.2 Hz, 3H).*

**Example 2. Methacrylic derivative of ketoprofen monomer (HKT)**

The synthesis of the methacrylic derivatives of ketoprofen (HKT) was carried out through a carbodiimide mediated Steglich esterification reaction **(Scheme 1)** as previously described by our group for the methacrylic derivative of ibuprofen and naproxen (Macromol. Biosci. 2013, 13, (9)). Briefly, KT (1 equiv) was dissolved in dichloromethane and subsequently, purified hydroxyethyl methacrylate (HEMA, 1 equiv) and 4-dimethylaminopyridine (DMAP, 0.1 equiv) were also added to the reaction mixture. DCC (1 equiv) was then added dropwise under continuous stirring and $N_2$ (g) atmosphere. The resultant solution was stirred overnight at room temperature. The reaction product was filtered to eliminate *N,N'*-diciclohexylurea (DCU) formed salt and the organic phase was washed with water and a saturated solution of $NaHCO_3$. Formerly, the resultant solution was dried with a saturated NaCl solution and anhydrous $MgSO_4$ and finally, DCM was evaporated under reduced pressure. The structure and purity of the product was determined by proton nuclear magnetic resonance ($^1$H-NMR) performed in a Varian Mercury 400 MHz equipment using $CDCl_3$ as a solvent at 25 °C **(Figure 3).**
***HKT*** *1H NMR (400 MHz, CDCl3) $\delta$H 7.76 - 7.65 (m, 3H), 7.60 (dt, J = 7.6, 1.5 Hz, 1H), 7.57 - 7.31 (m, 5H), 5.96 - 5.91 (m, 1H), 5.45 (p, J = 1.6 Hz, 1H), 4.35 - 4.17 (m, 4H), 3.76 (q, J = 7.2 Hz, 1H), 1.80 (t, J = 1.3 Hz, 3H), 1.47 (d, J = 7.2 Hz, 3H).*

**Example 3. Synthesis and characterization of copolymers of naproxen-based methacrylic monomer and 1-vinyl imidazole, poly(HNAP-co-VI)**

Copolymeric systems based on HNAP were synthesized by free radical polymerization in solution using 1-vinyl imidazole (VI) as co-monomer **(Scheme 1).**

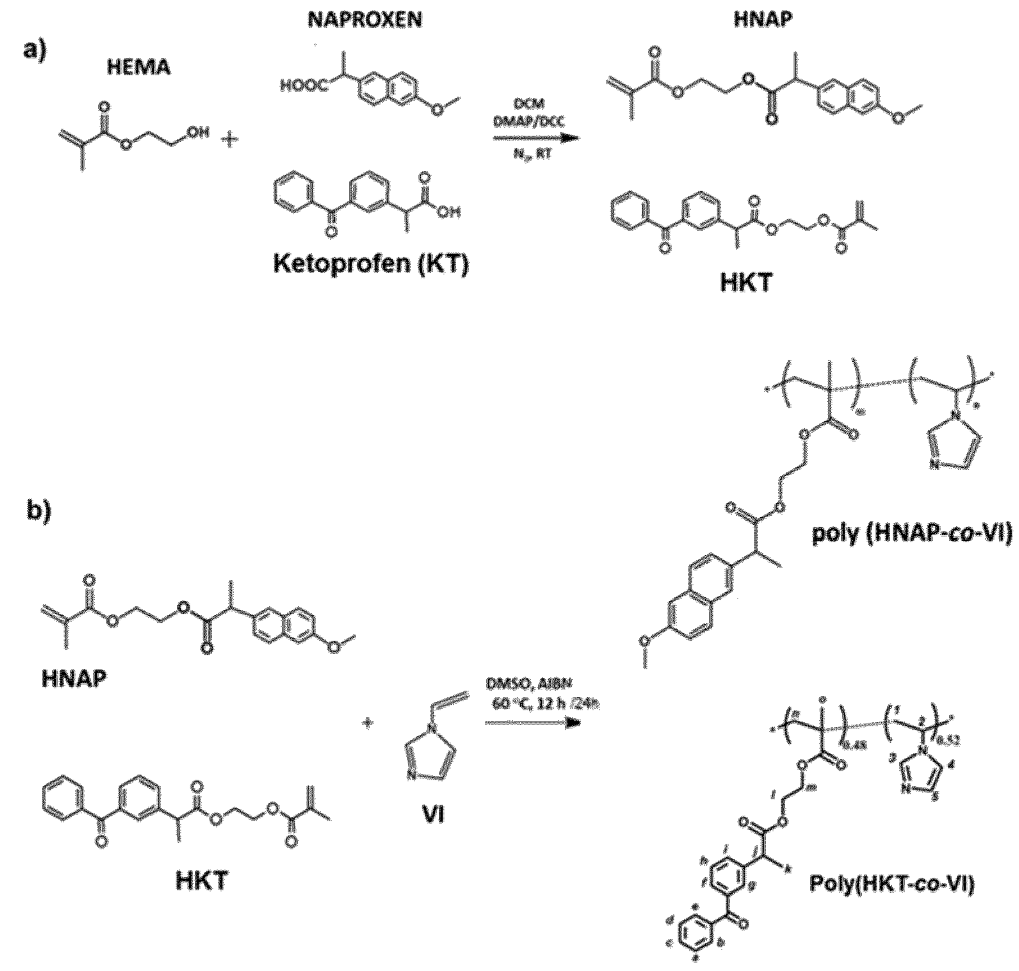


**Scheme 1.** Synthesis of a) the hydrophobic methacrylic derivative of naproxen (HNAP) or ketoprofen (HKT) through a carbodiimide mediated Steglich esterification reaction and; b) the copolymer poly(HNAP-*co*-VI) or poly(HKT-*co*-VI) via free radical polymerization reaction in solution.

A feed molar ratio HNAP:VI of 50:50, and an initial monomer concentration of 0.5 M were used in order to obtain polymers with hydrophilic-hydrophobic balance and molecular wieght suitable for self-assembly into NPs in aqueous media. The copolymers were named HNAP followed by their molar content in this monomer. Particularly, comonomers were dissolved in DMSO and the reaction mixtures were deoxygenated with $N_2$ (g) for 10 minutes. Then, AIBN ($1.5 \times 10^{-2} M$) was added as free radical initiator to the solution and the reaction vessel was transferred to an oven at 60 °C for 12 hours with the aim to thermally induce the polymerization and obtain high conversion copolymers. Copolymerization mixtures were purified by dialysis (Spectrum Laboratories, 3.5KDa molecular weight cut-off) against distilled water for 72 hours in order to eliminate residual unreacted monomer and low molecular weight species. Copolymers were isolated by freeze-drying as a white powder.

HNAP was successfully co-polymerized with VI by free radical polymerization as confirmed by the disappearance of the $^1$H-NMR signals corresponding to the vinyl protons of both monomers ($CH_{2\text{-}VI}$ at 4.9 and 5.45 ppm and $CH_{2\text{-}HNAP}$ at 5.6 and 5.9 ppm), the new $^1$H-NMR signals resultant from the methylene protons of the backbone chains ($CH_{3\text{-}m}$, $CH_{2\text{-}l}$ and $CH_{2\text{-}n}$ between 0.1 and 2.8 ppm) and the broadening of the signals as a result of the macromolecular nature of the copolymer **(Figure 2).** The copolymer molar composition was quantitatively determined from their corresponding $^1$H-NMR spectra by considering the signals between 0.1 and 2.8 ppm assigned to 8 protons of HNAP ($CH_{3\text{-}m}$, $CH_{3\text{-}i}$ and $CH_{2\text{-}l}$) and 2 protons of VI ($CH_{2\text{-}n}$) and the signals between 6.5 and 8.0 ppm resultant from 6 aromatic protons of HNAP ($CH_{\text{-}b\text{-}g}$) and 3 aromatic protons of VI (CH-p,q,r).

**p(HNAP-co-VI) (71-29; HNAP-71):** *$^1$H NMR (400 MHz, DMSO$_{d6}$) $\delta_H$ 7.89-6.45 (m, 9H (3VI + 6HNAP)), 4.53-3.39 (m, 8H (8HNAP)), 3.22-2.73 (s, 1H (1VI)), 2.28-0.10 (m, 10H (2VI + 8HNAP))*

HNAP feed molar composition ($F_{HNAP}$), homopolymer and copolymer HNAP molar composition ($f_{HNAP}$), reaction yields, average molecular weight ($M_w$), polydispersity index (*Đ*) and glass transition temperature ($T_g$) of the synthesized copolymers are summarized in **Table 2.**

**Table 2.** Summary of the physicochemical properties of newly synthesized HNAP71 and HKT48 copolymers

| $^1$H-NMR | | SEC | | | DESC | |
|---|---|---|---|---|---|---|
| **Sample** | **$F_{HNAP/HKT}$[a]** | **$f_{HNAP/HKT}$[b]** | **$M_w$ (KDa)[c]** | **Đ[d]** | **$T_{ge}$ (°C)** | **Yield[f](%)** |
| Poly **(HNAP-*co*-**VI) **HNAP71*** | 0.50 | 0.71 | 110 | 2.4 | 67 | **84** |
| Poly (**HKT**-*co*-VI) **HKT48** | 0.40 | 0.48 | 99 | 2.3 | 54 | **84** |
| *[a]Molar fraction of HKT/HNAP in the feed, [b]molar fraction of HKT/HNAP in the copolymer obtained by $^1$H-NMR, [c]Mol ecular weight and [d]Polidispersity of molecular weight distribution(Đ) obtained by SEC, [e]glass transition temperature ($T_g$) obtained by DSC and, percentage of the initial mass of monomers incorporated to the copolymer.* | | | | | | |

The polymerization successfully occurred. The differences among copolymer HNAP molar content ($F_{HNAP}$) and feed HNAP molar content ($f_{HNAP}$) can be attributed to the differences on the chemical reactivity of the monomers , and because total conversion of the polymerization was not reached. Polydispersity index values and yields approaches to the typical ones for a conventional radical polymerization process. According to the obtained reactivity ratios (see next section) a pseudoblock microstructure was obtained, however only one $T_g$ between the range of the $T_g$ values of both homopolymers (*i.e.* pHNAP and pVI) was detected for copolymers. This result indicates that microphase segregation was not produced or was not detected by the applied DSC method.

**Example 4. Synthesis and characterization of copolymer of ketoprofen-based methacrylic monomer and 1-vinyl imidazole, poly(HKT-co-VI)**

The copolymer based on HKT and VI was prepared via free radical polymerization **(Scheme 1)** a 0.4 feed molar content in HKT and 0.5 M initial monomers concentration were used. The copolymer was named HKTX being X the molar content in HKT. As a summary, both monomers were dissolved in DMSO and the reaction mixtures were deoxygenated with $N_2$ (g) for 10 minutes. Then, AIBN ($1.5 \times 10^{-2}$ M) was added and the reaction was kept at 60 °C for 12 hours. Copolymerization mixtures were dialyzed (Spectrum Laboratories, 3.5K molecular weight cut-off) against distilled water for 72 hours. The resultant copolymer was isolated by freeze-drying as a white powder.

HKT was successfully co-polymerized with VI by free radical polymerization as confirmed by the disappearance of the $^1$H-NMR signals corresponding to the vinyl protons of both monomers ($CH_{2\text{-}VI}$ between 4.5 and 5.0 ppm and $CH_{2\text{-}HKT}$ between 5.0 and 6.0 ppm), the new $^1$H-NMR signals resultant from the methylene protons of the backbone chains ($CH_{3\text{-}m}$, $CH_{2\text{-}l}$ and $CH_{2\text{-}n}$ between 0.1 and 2.8 ppm) and the broadening of the signals as a result of the macromolecular nature of the copolymer **(Figure 4).** The obtained copolymer is labeled according to their molar content in HKT obtained by $^1$H-NMR and its chemical structure is illustrated in **Figure 3.**

**poly(HKT-co-VI)(48:52) (HKT-48):** *$^1$H NMR (400 MHz, DMSOd6) $\delta$H 7.95-6.45 (m, 12H (3VI + 9HKT)), 4.53-3.39 (m, 5H (5HKT)), 3.22-2.73 (s, 1H (1VI)), 2.28-0.10 (m, 10H (2VI + 8HKT))*

The copolymers molar composition was quantitatively determined from their corresponding $^1$H-NMR spectra by considering the signals between 0.1 and 2.8 ppm assigned to 8 protons of HKT ($CH_{3\text{-}k}$, $CH_{3\text{-}o}$ and $CH_{2\text{-}n}$) and 2 protons of VI ($CH_{2\text{-}1}$) and the signals between 6.5 and 8.0 ppm resultant from 9 aromatic protons of HKT ($CH_{\text{-}a\text{-}i}$) and 3 aromatic protons of VI ($CH_{\text{-}3,4,5}$). For comparative purposes, average copolymers molar composition, reaction yields, molecular weights ($M_n$ and $M_w$), polydispersity indexes ($M_w/M_n$) and glass transition temperatures of the copolymer HKT48 and, previously described, HNAP71 are summarized in **Table 2.**

The differences among copolymer HKT molar content ($F_{HKT}$) and feed HKT molar content ($f_{HKT}$) that was also observed in case of HNAP 71 copolymer is explained by the differences on the reactivity ratios of the monomers, and because the yield was not 100%. HNAP71 has 10 KDa higher molecular weight when compared to HKT48 because, although HKT has higher molecular weight than HNAP (i.e. 366KDa and 277KDa, respectively), the copolymer molar content in HKT is approximately 2/3 that of HNAP. However, the fact that HKT has higher molecular weight makes this difference not significant when computing the mass of drug on each copolymer **(table 3).** Polydispersity index values and yields approach to the typical ones for a conventional radical polymerization reaction. HKT48 presented a unique glass transition temperature ($T_g$) indicating the homogeneous distribution of the copolymer chains.

**Example 5. Determination of Reactivity Ratios. Microstructural Analysis**

Reactivity ratios are kinetic parameters which measure the relative rate of addition of each comonomer to a growing copolymer chain and they allow the estimation of the copolymer average composition and the monomer sequence distribution. Those comonomers that differ in hydrophilicity and present very different reactivity ratios give rise to amphiphilic gradient microstructure by free radical copolymerization. Accordingly, the most reactive one is incorporated first and the least reactive one is incorporated at the last stages of the copolymerization reaction. These copolymers self-assemble in aqueous media leading to supramolecular structures (e.g. NPs). Reactivity ratios of the co-monomers ($r_{HNAP}/r_{HKT}$ and $r_{VI}$) were determined by in situ $^1$H-NMR monitorization of the copolymerization reaction at three different feed molar compositions by following the disappearance of the vinyl protons signals of both co-monomers, as previously described by Aguilar et al (Aguilar, M. R.; Gallardo, A.; Fernández, M. d. M.; Roman, J. S., Macromolecules 2002, 35, (6), 2036-2041). The data obtained were fit to a well-known integrated form of the differential copolymer equation defining the terminal model:

$$[HNAP]_t = [HNAP]_o \times \left(\frac{[VI]_t/[HNAP]_t}{[VI]_o/[HNAP]_o}\right)^{\frac{r_{VI}}{1-r_{VI}}} \times \left(\frac{1-r_{VI}+(r_{HNAP}-1)[VI]_t}{1-r_{VI}+(r_{HNAP}-1)[VI]_o}\right)^{\frac{(r_{HNAP}r_{VI})-1}{(1-r_{HNAP})(1-r_{VI})}}$$

or

$$[HKT]_t = [HKT]_o \times \left(\frac{[VI]_t/[HKT]_t}{[VI]_o/[HKT]_o}\right)^{\frac{r_{VI}}{1-r_{VI}}} \times \left(\frac{1-r_{VI}+(r_{HKT}-1)[VI]_t}{1-r_{VI}+(r_{HKT}-1)[VI]_o}\right)^{\frac{(r_{HKT}r_{VI})-1}{(1-r_{HKT})(1-r_{VI})}}$$

Being $[M]_t$ and $[M]_o$ the molar concentration of monomer *M* at time *t* and the initial monomer concentration, respectively; and $r_M$ the reactivity ratio of monomer *M.*

For the HNAP monomer, the obtained reactivity ratios were $r_{HNAP}$ = 3.16 ± 1.2 and $r_{VI}$ = 0.31 ± 0.06 meaning that active radicals incorporate HNAP monomer around 10 times faster than VI. These values were consistent with previously reported copolymerization reactions between VI and methacrylic derivatives using AIBN as free radical initiator and they revealed that the desired gradient microstructure is obtained. **Figure 5** shows the surface copolymerization diagram representing the instantaneous HNAP incorporation to the growing chain as a function of conversion and HNAP feed molar composition. Macromolecules rich in the most reactive monomer (HNAP) are formed at low conversions, and after most HNAP is consumed, VI-rich copolymer chains are formed. Few chains with intermediate compositions are obtained.

For the KHT monomer, the obtained reactivity ratios were $r_{HKT}$ = 3.74 ± 1.2 and $r_{VI}$ = 0.05 ± 0.06 meaning that active radicals incorporate HKT monomer around 100 times faster than VI. These values were consistent with previously reported copolymerization reactions between VI and methacrylic derivatives using AIBN as free radical initiator and they revealed that the desired gradient microstructure is obtained. **Figure 6** shows the surface copolymerization diagram representing the length of VI blocks as a function of conversion. It can be clearly seen that the length of VI blocks increases with conversion and that the higher the feed molar contents in HKT the higher the conversion degree needed to obtain longer hydrophilic VI-enriched segments.

**Example 6. NPs characterization for HNAP71-NPs**

NPs were obtained by nanoprecipitation method in aqueous media and they were labeled as the copolymers that were used in their preparation. Hydrodynamic properties such as mean hydrodynamic diameter ($D_h$, by intensity) and polydispersity index (PdI) or zeta potential (ξ) values were also obtained by DLS or LDE, respectively; and are summarized in **table 3.**

**Table 3.** Summary of hydrodynamic properties and encapsulation efficiency of newly prepared HKT48-based unloaded and Dx-loaded NPs (i.e. KT NPs and 14Dx-KT NPs, respectively), and HNAP71-based unloaded and Dx-loaded NPs (i.e. NAP NPs and 8Dx- NAP NPs, respectively).

| Sample | % EE | % LC | $[NPs]_{PCR}$ | $[NSAID]_{PCR}$ | $[Dx]_{PCR}$ | $D_h$ (nm) | PdI | ξ(mV) |
|---|---|---|---|---|---|---|---|---|
| **NAP NPs** | | | **45 ug/mL** | **27 ug/mL** | | 131 ± 3 | 0.115 ± 0.016 | + 29 ± 1 |
| **8Dx-NAP NPs** | 8 | 2.00 | **45 ug/mL** | **27 ug/mL** | **1 ug/mL** | 149 ± 2 | 0.085 ± 0.018 | + 26 ± 1 |
| **KT NPs** | - | | **45 ug/mL** | **24 ug/mL** | **-** | 117 ± 1 | 0.139 ± 0.004 | + 30 ± 1 |
| **14Dx-KT NPs** | 14 | 3.85 | **45 ug/mL** | **24 ug/mL** | **2 ug/mL** | 140 ± 1 | 0.081 ± 0.010 | + 29 ± 1 |

HNAP71-based NPs showed spherical morphology **(Figure 7),** a hydrodynamic diameter of 140 nm (*i.e.* between 100 and 200 nm), and positive surface charge (ξ = +28.8). Therefore HNAP71 NPs presented suitable hydrodynamic properties for passive accumulation and improved retention at inflamed areas according to bibliography.

NP diameter slightly varied (*i.e.* ± 5-10 nm) with [NPs] on the aqueous phase or with final volume keeping the values within the ranges that facilitate their sequestration by inflammatory cells. However, the particles diameter significantly increased up to 20 nm when the concentration of copolymer in the organic phase changes from 10 mg/mL to 15 mg/mL **(Table 4).**

**Table 4.** Hydrodynamic properties of nanoparticles based on HNAP71 at different final concentration of nanoparticles in the aqueous phase and at different final volumes.

| $[NPs]_{A.P.}$ [a] (mg/mL) | $V_F$ (mL) | $[HNAP71]_{O.P.}$ [b] (mg/mL) | $D_h$[c] (nm) | PdI[d] | ξ[e] (mV) |
|---|---|---|---|---|---|
| 1.0 | 10 | 10 | 140.2 ± 2.1 | 0.181 ± 0.002 | 28.8 ± 0.7 |
| | 20 | 10 | 126.6 ± 1.4 | 0.186 ± 0.021 | 27.8 ± 0.1 |
| | 30 | 10 | 132.1 ± 1.6 | 0.173 ± 0.023 | 30.6 ± 1.4 |
| 5.0 | 10 | 10 | 129.5 ± 0.2 | 0.164 ± 0.025 | 31.2 ± 1.5 |
| | 20 | 10 | 131.7 ± 1.3 | 0.165 ± 0.021 | 29.3 ± 0.8 |
| | 30 | 15 | 150.0 ± 2.9 | 0.197 ± 0.016 | 28.8 ± 1.2 |

*[a]Concentration of NPs in the aqueous phase and [b]concentration of the copolymer in the organic phase [c]hydrodynamic diameter, [d]polydispersity and [e]zeta potential*

VI ionizable groups played a key role in the NP stability as a function of pH. **Figure 8a** shows the size distribution and the ξ values of the HNAP71 nanoparticulated system at different *pH.* When *pH* 5.0 is reached, it is observed a broadening of the size distribution and a significant increase in the intensity of the peak in the microscale suggesting the agglomeration of NPs that may be attributed to the decrease in the surface charge and, hence, to the reduction of the electrostatic repulsion between particles as the *pH* approaches to the $pK_b$ of VI ($pK_b$ = 5.0-6.0). The phenomenon is partially reversible as when pH is reduced to 4.0 original hydrodynamic properties are recovered. Based on these results and on the previously reported strong buffering capacity of VI, it was necessary the use of 0.1*M* Acetic Acid to keep pH value constant avoiding NPs agglomeration and maintaining their stability over time. **Figure 8b** shows that there are no significant changes on the hydrodynamic properties of HNAP71 NPs obtained at pH 4 and stored during 4 weeks at 4°C. Furthermore, NPs recover their initial hydrodynamic properties after freeze-drying and dispersion in the buffer solution at *pH* 4.0 only if they are sonicated for 10 minutes with an ultrasound tip (30 % amplitude) **(Figure 8c).** Therefore, size and morphology of the particles could be modulated by controlling the molar composition of the copolymers and the concentration of copolymer in the organic phase and NPs can be stored at 4 °C and pH 4.0 for at least one month or in powder.

**Example 7. NPs characterization for KHT48-NPs**

If the hydrophobic-hydrophilic balance is suitable, the pseudo-gradient microstructure of HKT48 may self-assemble into nanoparticles in aqueous media having a hydrophobic core containing covalently linked KT and a hydrophilic shell formed by VI. Protonable amine groups of VI should be exposed on the NPs surface providing the particles with the desired positive surface charge to favor internalization by inflammatory cells. To confirm that, NPs were prepared by nanoprecipitation method in aqueous media as previously described and they were extensively characterized. NPs were labeled as the copolymer that was used in their preparation. SEM micrograph of the NPs confirmed the successful NPs formation presenting spherical shape, slight polydispersity in size, and sizes of about 100 nm **(Figure 9).** Hydrodynamic properties such as hydrodynamic diameter ($D_h$, by intensity) and polydispersity index (*PdI*) or zeta potential ($\xi$) values were also obtained by DLS or LDE, respectively. In order to compare with previously described HNAP71 NPs, current and previous results are summarized in **Table 3.**

Positive $\xi$ values confirmed the presence of VI protonable amine groups on the surface of the NPs as indicated by the positive surface charge. Moreover, the $\xi$ values higher than + 30 mV are indicative of good stability in suspension. The spherical morphology, positive surface charge and diameters between 100 and 200nm makes HKT48-based NPs suitable for passive accumulation at inflamed areas when administered systemically[27, 28] as well as for an improved sequestration by inflammatory cells avoiding lymphatic drainage. When comparing with HNAP71-based NPs, they present a significantly smaller diameter (i.e. 20 nm smaller than HNAP71 NPs) but not significant differences in surface charge probably because of the saturation of the surface with the amine groups of VI. PdI values are below 0.2 in all cases.

As occurred with NAP NPs, the diameter or surface charge of KT NPs nanoparticulated systems did not vary with the final concentration on the aqueous phase or with the final volume. The key variable is again the concentration of copolymer in the organic phase which when increased lead to NPs with 50 nm larger diameter **(Table 5).**

**Table 5.** Hydrodynamic properties of nanoparticles based on HNAP71 at different final concentration of nanoparticles in the aqueous phase and at different final volumes.

| $[NPs]_{A.P.}$ $^{a}$ | $V_F$ (mL) | $[HKT48]_{O.P.}$ $^{b}$ | $D_h$ $^{c}$ (nm) | PdI$^{d}$ | $\xi^{e}$(mV) |
|---|---|---|---|---|---|
| 1.0 mg/mL | 10 | 10 mg/mL | 117±1 | 0.139 ± 0.004 | + 29 ± 1 |
| | 20 | 10 mg/mL | 119 ± 3 | 0.153 ± 0.018 | + 29 ± 1 |
| | 30 | 10 mg/mL | 116 ± 1 | 0.142 ± 0.027 | + 30 ± 1 |
| 5.0 mg/mL | 10 | 10 mg/mL | 115 ± 1 | 0.124 ± 0.008 | + 30 ± 1 |
| | 20 | 10 mg/mL | 119 ± 1 | 0.112 ± 0.013 | + 31 ± 1 |
| | 30 | 15mg/mL | 163 ± 3 | 0.186 ± 0.021 | + 29 ± 1 |

Finally, to determine the most suitable conditions for NPs storage, a pH study, stability in suspension study and freeze-drying study were performed. **Figure 10a** shows the size distribution and the $\xi$ values of the KT NPs at pH values 4.0, 4.5, 5.0 and 5.5. *pH* 5.5 was set as aggregation pH as it was observed a broadening of the size distribution and an increase in the intensity of the peak in the microscale which corresponds to agglomerated NPs. A decrease in surface charge was observed as pH increases due to deprotonation of amine groups of VI as the *pH* approaches to the $pK_b$ of VI ($pK_b$ = 5.0-6.0). The reduction in the electrostatic repulsion between particles caused the increase in NPs agglomeration. Therefore, as previously described for naproxene-based NPs, it was necessary the use of 0.1 *M* Acetic Acid to maintain the stability over time. **Figure 10b** shows that, under these conditions, there are no significant changes on the hydrodynamic properties of HKT48 NPs at 0 days, 14 days and 28 days when stored at 4 °C. Furthermore, NPs recover their initial hydrodynamic properties after freeze-drying and dispersion in the buffer solution at *pH* 4.0 when sonicated for 10 minutes with an ultrasound tip (30 % amplitude) **(Figure 10c).** Therefore, as for its NAP-containing analogue, the concentration of copolymer in the organic phase and NPs can be stored at 4 °C and *pH* 4.0 for at least one month or in powder.

**Example 8. Dexamethasone loaded NPs**

Dexamethasone (Dx) was encapsulated to assess if the combination of Dx with NAP or KT leads to the *IL12b* synergistic inhibition. To achieve the maximum final concentration of Dx in our systems we performed a screening of different % w/w with respect to the copolymer (5% w/w, 10% w/w, 15% w/w and 20% w/w) and the final mass of Dx encapsulated was computed by HPLC **(Figure 11).** It is clearly observed a more efficient encapsulation when using HKT48 copolymer to prepare the NPs. This result might be explained because of the higher polarity of Dx that will

facilitate its encapsulation in HKT48, which has more VI (hydrophilic) content. The % w/w chosen as the preferred one was 15% w/w because the maximum mass of Dx was achieved (%EE = 14). The amount of drug encapsulated correlates with the increase in diameter of the nanoparticles, the increase is higher in Dx-loaded systems when using HKT48 than when using HNAP71 **(Table 2).** 14Dx-HKT48 is 10 nm smaller than 8Dx-HNAP71 but no significant differences are observed in $\xi$ values. Therefore, for a given concentration, KT NPs allow to duplicate the amount of Dx encapsulated when compared to HNAP71-based systems. NPs are labeled as XDx-NAP/KT NPs being *X* the encapsulation efficacy.

**Example 9. *In vitro* cytotoxicity assay of NPs (naproxen)**

Cytotoxicity of 8Dx-NAP NPs was studied and compared with unloaded NAP NPs and the free dexamethasone at a concentration equal to the dexamethasone encapsulated in 8Dx-NAP NPs (*i.e.* 56.66 $\mu$M of dexamethasone per 1 mg/mL of NPs) using RAW264.7. None of the assayed *NP or dexamethasone concentrations* resulted cytotoxic for RAW264.7 after 24 h as cell viability was always higher than 70 % (ISO 10993-5:2009). 8Dx-NAP NPs (dashed white) and unloaded naproxen NPs (black) formulations showed non-statistically significant differences (*$p < 0.05$) with respect to the control (CP) at any of the tested concentrations but the highest one (0.25 mg/mL) in case of naproxen NPs and the two highest ones (0.25 and 0.125 mg/mL) in case of 8Dx-NAP NPs. However, free drug (red) presented significant differences with the control at all tested concentrations. These results suggest a reduction of the cytotoxicity of encapsulated dexamethasone when compared to free drug **(Figure 12)** although no statistically significant differences are observed after 24h of incubation.

**Example 10. *In vitro* cytotoxicity assay of NPs (ketoprofen)**

Cytotoxicity of free Dx (maximum concentration encapsulated), 14Dx-KT NPs and unloaded KT NPs were tested using murine macrophages (RAW264.7) as model inflammation-related cells. **Figure 13a** shows the cell viability compared to a control of untreated cells. None of the concentrations tested showed cytotoxicity after 24 h as cell viability was always higher than 70 % (ISO 10993-5:2009). There were no statistically significant differences (*$p < 0.05$) between Dx-loaded and unloaded NPs (i.e. KT NPs-14Dx and KT NPs) or between free Dx and encapsulated Dx (i.e. free Dx and KT NPs-14Dx) at any of the concentrations tested. When comparing KT-based systems with previously described NAP-based systems **(Figure 13a and 13b),** unloaded NAP NPs presented lower toxicity than KT NPs probably because of the slower internalization **(Figure 13a)** whereas Dx-loaded systems showed non-significant differences (*$p < 0.05$) in terms of cytotoxicity. Therefore, NAP-based or KT-based Dx-loaded systems presented cytotoxicity similar to free Dx meaning that toxicity of Dx dominates but does not sum up to the intrinsic toxicity of the nanoparticulated system in which it is incorporated.

**Example 11. Nitric Oxide (NO) assay (naproxen)**

NO at appropriate physiological levels is a molecule of key importance for the vascular system and a mediator of immunity and inflammation. However, NO overproduction by $M_1$ macrophages in response to an inflammatory stimuli causes oxidative stress, chronic inflammation and, ultimately, tissue damage. NO release was evaluated using Griess reagent in order to determine the anti-inflammatory capacity of the developed formulations. Basically, during the Griess reaction dinitrogen trioxide ($N_2O_3$) generated from the acid-catalyzed formation of nitrous acid from nitrite (or autoxidation of NO) reacts with sulfanilamide to produce a diazonium ion which is then coupled to *N*-(1-napthyl) ethylenediamine to form a chromophoric azo product that absorbs strongly at 540 nm. LPS-induced NO release was measured after 24 h of LPS administration and challenging with different concentrations of 8Dx-NAP NPs, unloaded naproxen NPs or free dexamethasone **(Figure 15).**

After 24 h of incubation, free dexamethasone and 8Dx-NAP reduced NO release in a statistically significant manner at all tested concentrations whereas unloaded naproxen NPs counteract LPS-induced NO release in a statistically significant (*$p < 0.05$) way with respect to LPS treated cells (LPS+) at all concentrations but 0.09 and 0.045 mg/mL **(Figure 15).** At NPs concentration between 0.125 and 0.023 mg/mL, it is observed a statistically significant (#$p < 0.05$) reduction of NO released levels when comparing unloaded NAP NPs and 8Dx-NAP NPs. Moreover, for this range of concentrations 8Dx-NAP NPs and free Dexamethasone present non-significant differences in terms of reduction of % NO-release. These results demonstrate that encapsulation does not diminish dexamethasone capacity to reduce NO-release levels at NPs concentrations between 0.125 and 0.023 mg/mL. Moreover, an additive but not synergistic effect is observed on the reduction of NO released after 24 hours of macrophages LPS-activation when encapsulating Dexamethasone in NAP-containing NPs. A concentration of NP of 0.045 mg/mL was chosen for further experiments as it was the lowest non-toxic concentration of NPs in which most significant differences between unloaded NPs and dexamethasone-loaded NPs were observed.

**Example 12. Reverse transcription, real-time quantitative PCR (RT-qPCR) analysis (naproxen)**

RT-qPCR analysis of the transcript levels of the main markers of inflammatory-autoimmune diseases like CIA (i.e. *IL12b, IL23a* and *TNF-$\alpha$*) was performed in order to identify the key molecular players on the previously described dexamethasone dose-sparing capacity of NAP. The transcript levels of these markers were analyzed by means of the primers shown in table 1, previously described in materials and methods. First of all, the transcript levels were assessed under non-activated normal tissue conditions. It has been reported that positively charged NPs could induce $M_1$-like polarization through toll-like receptor 4 (TLR4) signaling and cause toxicity by interacting with cellular components. Hence, treatment with cationic NPs leads to overexpression of *IL12b, IL23a,* and *TNF-$\alpha$* $M_1$ markers which are the same as those related to pathogenesis in CIA rats (*i.e. IL12b, IL23a,* and *TNF-$\alpha$*). Therefore, to check if the system itself lead to M1-polarization, expression of these specific gene markers by unstimulated RAW264.7 cells after treatment with either free dexamethasone, unloaded naproxen-bearing nanoparticles (NAP NPs) or dexamethasone-loaded naproxen-bearing nanoparticles (8Dx-NAP NPs) was determined. Additionally, extensively reported Dexamethasone capacity to polarize macrophages to $M_2$ phenotype at concentrations below 100 nM and anti-angiogenic properties at higher concentrations justified the study of $M_2$ phenotype and angiogenesis markers expression after treatment with either free dexamethasone or 8Dx-NAP NPs in normal tissue conditions. **Figure 16a** shows the transcript levels of *TNF-$\alpha$, IL12b* and *IL23a* ($M_1$ markers) and *VEGF* (angiogenesis and $M_2$ marker), *TGF-$\beta$* and *IL10* ($M_2$ markers) determined by RT-qPCR after 1 day and 7 days of treatment. Results are expressed relative to the corresponding level of expression of each transcript in the untreated sample (*i.e.* only culture media). **Figure 16b** showed that after 24h of treatment with 8Dx-NAP NPs or naproxen NPs, all $M_1$ markers were under-expressed (green) or equally expressed (black) when compared to the untreated sample showing no $M_1$-polarization or cytotoxicity at this time point. These data correlate with the cytotoxicity results after 24h treatment. In contrast, all genes but *IL12b* were overexpressed (red) after 7 days of treatment with any of the nanoparticulated systems tested. The overexpression of *IL23a* was markedly lower in encapsulated Dexamethasone system when compared to its free form. This indicates that encapsulation improved biocompatibility by reducing the pro-inflammatory activity of free dexamethasone. Moreover, dexamethasone-loaded naproxen NPs also showed lower levels of *IL23a* when compared to unloaded NAP NPs probably because Dexamethasone is counteracting the minor long-term $M_1$-polarization caused by the positive surface charge of NPs. On the other hand, the *TNF-$\alpha$* increased levels after long-term treatment might be related with the presence of an NSAID in the system, an extensively reported effect related to important gastrointestinal side effects. Basically, NSAIDs counteract prostraglandin-E2 (PGE2) overproduction and hence, reduce PGE2 positive feedback on TNF-$\alpha$ levels. This effect was not observed after 24 hours of treatment as with other previously described free NSAIDs treatments. However, after 7 days, *TNF-$\alpha$* levels were increased in an evident and comparable manner for the three systems under study being slightly higher for the system combining NAP/Dx. Therefore, the linkage of the NSAID to the polymeric structure and its controlled release delays the onset of this response whereas Dexamethasone encapsulation did not counteract it. All in all, the most interesting result was the almost knockdown of *IL12b* expression when treating RAW264.7 macrophages with 8Dx-NAP NPs. Dexamethasone in its free form inhibited *IL12b* expression at both time points tested. NAP NPs also inhibit *IL12b* expression (1 day) or kept it at basal levels (7 days). However, there was a clear synergistic effect in the trans-repression of this gene when encapsulating Dexamethasone in naproxen-bearing NPs. It has been reported that *$IL12b^{-/-}$* mice are completely protected from CIA[50]. Therefore, if this synergistic repression of *IL12b* occurs also under inflammatory conditions might be the reason for the dexamethasone dose-sparing capacity of NAP in the treatment of this disease.

On the other hand, **Figure 16** shows that, as expected, no over-expression of $M_2$ markers is observed for either free Dexamethasone or encapsulated dexamethasone (2.55 $\mu$M) as the concentration of dexamethasone is higher than 100 nM which is the reported limit concentration for polarization. At concentrations higher than 100 nM, dexamethasone is expected to reduce *VEFG* expression, a major contributor to macrophage-derived angiogenic activity, and also *IL10* expression contributing to reduction of allergic inflammation in the treatment of allergic diseases. This anti-angiogenic capacity described for dexamethasone in several cell lines is clearly observed after 7 days when it is in its free form (free dexamethasone) but not when encapsulated (8Dx-NAP-NPs). The same occurs with the *IL10* transrepression which was clearly observed in dexamethasone on its free form at both time points studied but was not occurring when cells were treated with 8Dx-NAP-NPs instead. Therefore, encapsulation and controlled release of the drug or its combination with naproxen is causing retardation or suppression of anti-angiogenic activity and transrepression of *IL10.* The reduction of *IL10* repression caused by dexamethasone when combining it with NAP might be related to the observed synergistic repression of *IL12* as it has been reported that blockage of *IL10* translates into *IL12* overproduction.

Finally, as *IL12b* is primarily produced by activated macrophages, to confirm Dx/NAP synergistic effect under simulated inflammatory conditions, macrophages are polarized to $M_1$ pro-inflammatory phenotype by LPS-stimulation. At the same time, they are treated with either culture media (CP (+LPS)), free dexamethasone (2.55 $\mu$M), unloaded NPs (0.045 mg/mL) or dexamethasone-loaded NPs (2.55$\mu$M dexamethasone and 0.045 mg/mL NPs). Again, the expression of $M_1$ marker genes was expressed relative to the corresponding level of expression of each transcript in the untreated sample (**Figure 17**). When comparing to the inflamed control (+LPS), after 24 hours of treatment with naproxen NPs

transcript levels of pro-inflammatory cytokines were not altered. However, free dexamethasone and 8Dx-NAP NPs treatment significantly reverse $M_1$ polarization. Therefore, the short-term activity of the system is attributed to dexamethasone release. Furthermore, the reduction in pro-inflammatory cytokines transcript levels is less significant when treating with free dexamethasone meaning that encapsulation of dexamethasone improves anti-inflammatory behavior. Moreover, the system combining covalently linked NAP and physically entrapped dexamethasone (8Dx-NAP NPs) reduced in a highly significant manner the levels of *IL12b* at both time points reproducing the previously obtained results in normal tissue conditions. The significantly increased expression of *TNF-*$\alpha$ that is clearly observed in Dx/NAP combining system might also play a role on *IL12b* synergistic repression. Some authors reported that this increase in *TNF-*$\alpha$ is not necessarily pathogenic but it could be beneficial as it regulates adaptive immune response via inhibition of IL12 and IL23. These results support this affirmation and suggest that *TNF-*$\alpha$-induced inhibition of IL12 and IL23 occurs through repression of *IL12b* gene, a gene codifying for IL12-p40 subunit.

The study at the molecular level of the expression of specific markers of autoimmune diseases confirmed that the combination of naproxen and dexamethasone translates into a synergistic reduction of *IL12b* transcript levels either in normal or inflammatory conditions. *IL12b* gene transcribes for the IL12-p40 subunit that is common for IL12 and IL23. IL12 is decisive in regulating lineage commitment to T helper 1 cells (Th1) development, whereas IL23 is essential for the maturation and stability of IL17-producing T helper 17 cells (Th17). Therefore, Dx/NAP combination and, in particular, the newly described Dexamethasone -loaded NAP bearing NPs have potential for the treatment of diseases in which IL12 and IL23 are overexpressed and Th1 and Th17 play a key role in pathogenesis. That is the case of autoimmune/inflammatory disorders like Crohn's disease, ulcerative colitis, psoriatic arthritis, rheumatoid arthritis, human multiple sclerosis, or psoriasis. This combination of FDA-approved drugs acts at a genetic level inhibiting *IL12b* expression by macrophages, a IL12-p40 subunit codifying gene, and, hence, decreasing IL12 and IL23 levels. This could offer an alternative to an antibody (Sterala© (ustekinumab)) recently approved by the FDA for the treatment of Crohn's disease, psoriasis, psoriatic arthritis and plaque psoriasis. This monoclonal antibody blocks IL12-p40 subunit preventing IL12 and IL23 binding to their receptors.

**Example 13. *In vitro* anti-inflammatory activity of NPs. Nitric Oxide (NO) assay (ketoprofen)**

Anti-inflammatory capacity of the systems after 24 h and 48 h was assessed indirectly by measuring the levels of nitric oxide (NO) released by lipopolysaccharide (LPS)-activated macrophages as a fast and quick manner to select the most appropriate [NPs] to have anti-inflammatory effect. When RAW264.7 cells are activated by LPS, they polarize to their pro-inflammatory phenotype ($M_1$) and they start overproducing NO, a well-known inflammatory mediator. Therefore, a reduction of LPS-induced levels of NO can be interpreted as an indirect marker of anti-inflammatory activity. Levels of NO released was measured after 24 **(Figure 18,** *black)* and 48 hours **(Figure 18,** *white*) of incubation with LPS and either unloaded NAP/KT NPs **(Figure 18,** *upper row*) and loaded NAP NPs-8Dx or KT NPs-14Dx **(Figure 18,** *lower row*) at different concentrations. Results at different time points demonstrate that NAP or KT-based unloaded systems significantly (#$p < 10^{-5}$) improve their activity (i.e. reduce NO release) after 48 h when compared to 24 h whereas Dx-loaded systems does not improve after 24 h. This illustrates the faster anti-inflammatory activity of systems loaded with Dx which is physically entrapped and, hence, is released earlier than covalently linked NSAIDs. When comparing unloaded KT-based and NAP-based systems **(Figure 18,** *upper row*) there are no clear differences in terms of reduction of NO released levels at any of the two time points tested. However, improved behavior is observed when treating cells with KT NPs-14Dx in comparison to NAP NPs-8Dx **(Figure 18,** *lower row*). This might be attributed to the higher content in Dx (5.1uM and 2.55uM, respectively).

Moreover, **Figure 19** presents LPS-induced NO release after 48 h of LPS administration and challenging with different concentrations of unloaded NAP NPs/KT NPs (*black*) 8Dx-NAP NPs/14Dx-KT NPs (*white*), or free Dx (*red*). After 48 h of incubation, all systems counteract LPS-induced NO release in a statistically significant manner for all concentrations tested (#$p < 10^{-5}$) with respect to LPS treated cells (LPS+). Results demonstrate that Dx encapsulation does not compromise NO reduction capacity after 48h at any of the concentrations tested in case of KT-based systems. Moreover, Dx-loaded systems perform better than free Dx at specific concentrations of KT-based systems (*$p < 0.05$; [NPs] = 0.045 mg $mL^{-1}$, and 0.023 mg $mL^{-1}$). One of these concentrations, 0.045 mg $mL^{-1}$, was chosen for further RT-qPCR analysis as it is the maximum concentration of NPs with improved reduction of NO released levels and it will allow comparison with RT-qPCR results obtained for previously described NAP-based systems.

**Example 14. Reverse transcription, real-time quantitative PCR (RT-qPCR) analysis (ketoprofen)**

Antigen-presenting cells (macrophages and dendritic cells) are responsible for the secretion of IL12 and IL23 in response to external stimuli. In particular, macrophages polarize to $M_1$ pro-inflammatory phenotype and start secreting them to differentiate naive T cells. The positive surface charge of NPs can lead to M1 polarization of macrophages, which is also a marker of low biocompatibility. Therefore, it is necessary to understand how the system itself affects the

expression of the key players in autoimmune/inflammatory diseases (*i.e. IL23a, IL12b,* and *TNF-*$\alpha$) in normal tissue conditions. **Figure 20a** shows the transcript levels of *TNF-*$\alpha$*, IL-12b,* and *IL-23a* determined by RT-qPCR after 1 day and 7 days of treatment. Results are expressed relative to the corresponding level of expression of each transcript in the untreated sample (i.e. only culture media). The expression levels of these transcript were analyzed by means of the primers shown in table 1, previously described in materials and methods. The synergistic repression of *IL12b* was only observed for Dx-loaded NPs in short term (day 1) but, in any case, as significantly as for NAP/Dx combination. On the contrary, there was an increase in TNF$\alpha$ levels as expected because of the higher potency of KT. These results suggest that the there is no direct relationship between the increase in TNF-$\alpha$ and the repression in Il12b; and that combining Dx with other NSAIDs is not as effective as combining it with naproxen. To confirm these findings also under inflammatory conditions, macrophages are polarized to M1 pro-inflammatory phenotype by LPS-stimulation. At the same time, they are treated with either culture media (CP (+LPS)), free Dx (5.1 $\mu$M), unloaded NPs (0.045 mg/mL) or Dx-loaded NPs (5.1$\mu$M Dx and 0.045 mg/mL NPs). Again, the expression of M1 marker genes was expressed relative to the corresponding level of expression of each transcript in the untreated sample **(figure 20b).** Under inflammatory conditions the synergy is also observed but, in this case, after 7 days of treatment. Again, it was not as significant as when treating cells with the Dx/NAP combination. However, the KT systems either loaded with Dx or unloaded reduces expression of the three M1 markers in a highly significant manner than the NAP-based systems previously described. This result was expected as the system incorporated twice the amount of Dx, KT is a more potent NSAID than NAP in COX inhibition, and the internalization of this system was faster. In fact, unloaded KT-NPs showed better anti-inflammatory activity than Dx-loaded NPs in terms of improved reduction of the expression of M1 markers under inflammatory conditions. Therefore, although the combination Dx/KT is not as interesting as NAP/Dx, the KT-based system could be used as vehicle for other drugs in the treatment of inflammatory processes. Moreover, it can be concluded that there is no relationship between TNF-$\alpha$ levels or NSAID potency, and synergistic repression of Il12b when in combination with Dx.

## Claims

1. Polymeric nanoparticle comprising hydrophobic monomeric NSAID-based units, wherein the nanoparticle is loaded with a corticosteroid.

2. Polymeric nanoparticle according to claim 1 wherein the NSAID is selected from aspirin, diflunisal, indomethacin, suldinac, tolmetin, etodolac, ketorolac, diclofenac, fenclofenac, alcofenac, naproxen, ketoprofen, ibuprofen, flurbiprofen, fenoprofen, fenbufen, oxaprozin, tiaprofenic acid, mefenamics, meclofenamics, flufenamics, tolfenamics, and lumiracoxib.

3. Polymeric nanoparticle according to claim 2 wherein the NSAID is naproxen or ketoprofen.

4. Polymeric nanoparticle according to any preceding claim wherein the monomeric NSAID-based unit is selected from ketoprofen-based methacrylic monomer and naproxen-based methacrylic monomer.

5. Polymeric nanoparticle according to claim 4 wherein the monomeric NSAID-based unit is a naproxen-based methacrylic monomer.

6. Polymeric nanoparticle according to any preceding claim wherein the nanoparticle also comprises monomers selected from N-vinilpirrolidonavinylpyrrolidone, N,N-dimethyilacrilamidae, N-isopropilacrilamidaisopropylacrilamide, 2-hidroxitilmetacrilatohydroxyethyl metacrilate, 2-hidroxitilacrilatohydroxythyl acrilate, polyethylenglycol methacrylatemetacrilato de polietilenglicol, polyethylenglycol acrylateacrilato de polietilenglicol, metacrilato de 2-hidroxipropilohydroxypropil methacrylate, metacrilato de N-etilmorfolinaethylmorpholine methacrylate, N-ethylmorpholine acrylateacrilato de N-etilmorfolina, N-hydroxyethylpyrrolidone methacrylatemetacrilato de N-hidroxietilpirrolidona, N-hydroxyethylpyrrolidone acrylateacrilato de N-hidroxietilpirrolidona, 2-vinylpiridine 2-vinilpiridina and 1-vinyl imidazole.

7. Polymeric nanoparticle according to claim 6 wherein the monomer is 1-vinyl imidazole.

8. Polymeric nanoparticle according to any of claims 4 to 7 wherein the polymeric nanoparticles are copolymers which comprise monomeric units of naproxen-based methacrylic monomer or ketoprofen-based methacrylic monomer, and 1-vinyl imidazole.

9. Polymeric nanoparticle according to any preceding claim wherein the corticosteroid is physically entrapped in the polymeric nanoparticles.

10. Polymeric nanoparticle according to any preceding claim wherein the corticosteroid is selected from aclometasone, amcinonide, beclomethasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumethasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone furoate, paramethasone, prednicarbate, prednisone, prednisolone, prednylidene, remexolone, tixocortol, triamcinolone and ulobetasol, and combinations, pharmaceutically acceptable salts and esters thereof.

11. Polymeric nanoparticle according to claim 10 wherein corticosteroid is dexametasone.

12. A method for preparing the polymeric nanoparticles according to claim 1 which comprises the following steps:

(a) synthesizing of hydrophobic monomeric NSAID-based units, preferably the methacrylic derivative of the NSAID-based unit;
(b) obtaining a polymer by free radical polymerization in solution of the the NSAID-based unit from step (a) or optionally a copolymer by free radical polymerization in solution of the NSAID-based unit ofstep (a) and monomers selected from N-vinylpyrrolidone, N,N-dimethylacrilamide, N-isopropylacrilamide, 2-hydroxyethyl metacrilate, 2-hydroxythyl acrilate, polyethylenglycol methacrylate, polyethylenglycol acrylate, 2-hydroxypropil methacrylate, N-ethylmorpholine methacrylate, N-ethylmorpholine acrylate, N-hydroxyethylpyrrolidone methacrylate, N-hydroxyethylpyrrolidone acrylate, 2-vinylpiridine and 1-vinyl imidazole; and more preferably the monomer is 1-vinyl imidazole; and
(c) encapsulating a corticosteroid in the polymer or optionally the copolymer obtained in step (b).

13. Polymeric nanoparticles according to any of claims 1 to 11 for use as a medicament.

14. Polymeric nanoparticles according to any of claims 1 to 11 for use in the treatment of an autoimmune and/or inflammatory disease.

**15.** Polymeric nanoparticles for use according to claim 14 wherein the autoimmune-inflammatory disease is selected from Crohn's disease, ulcerative colitis, psoriatic arthritis, rheumatoid arthritis, ankylosing spondylitis, human multiple sclerosis, and psoriasis.

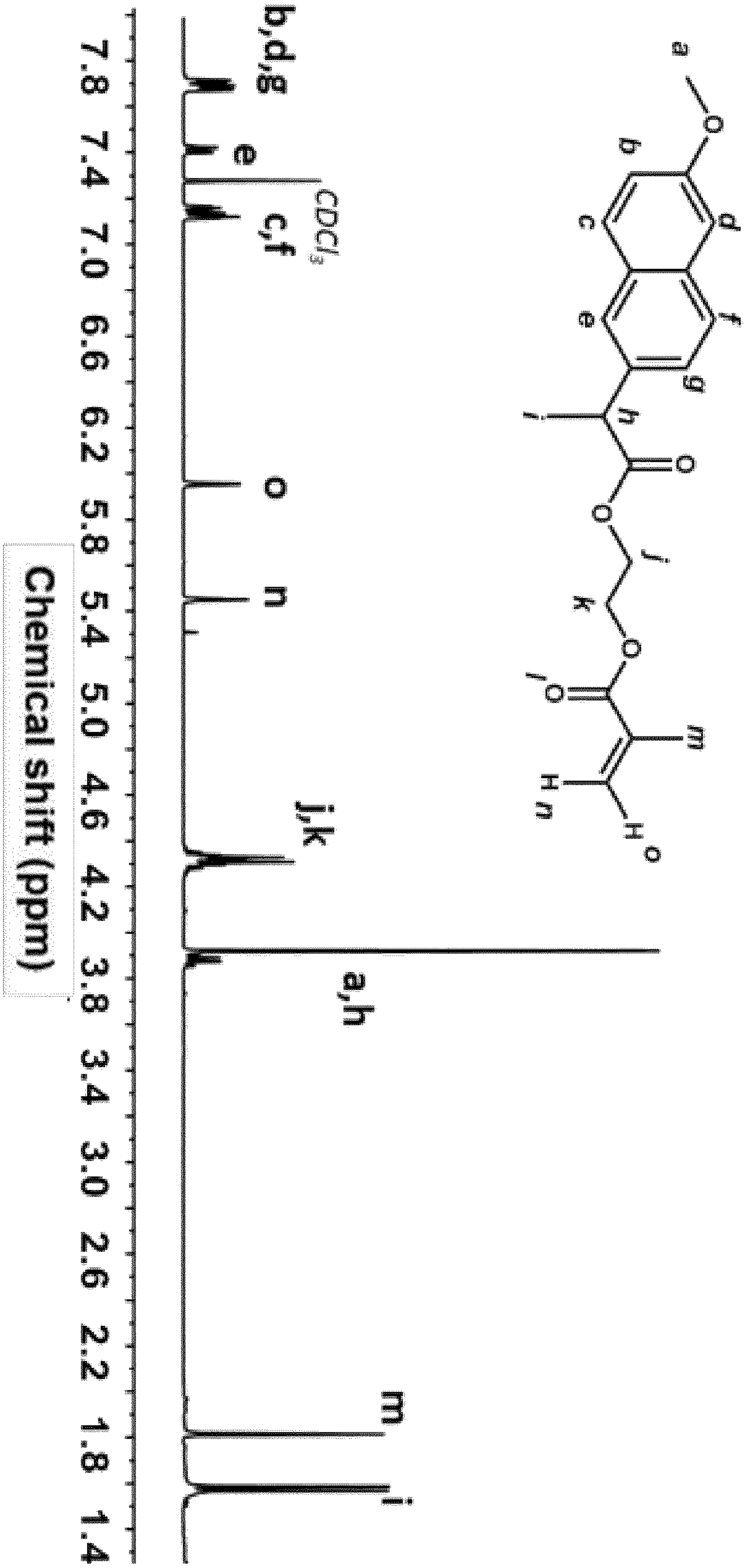


FIG. 1

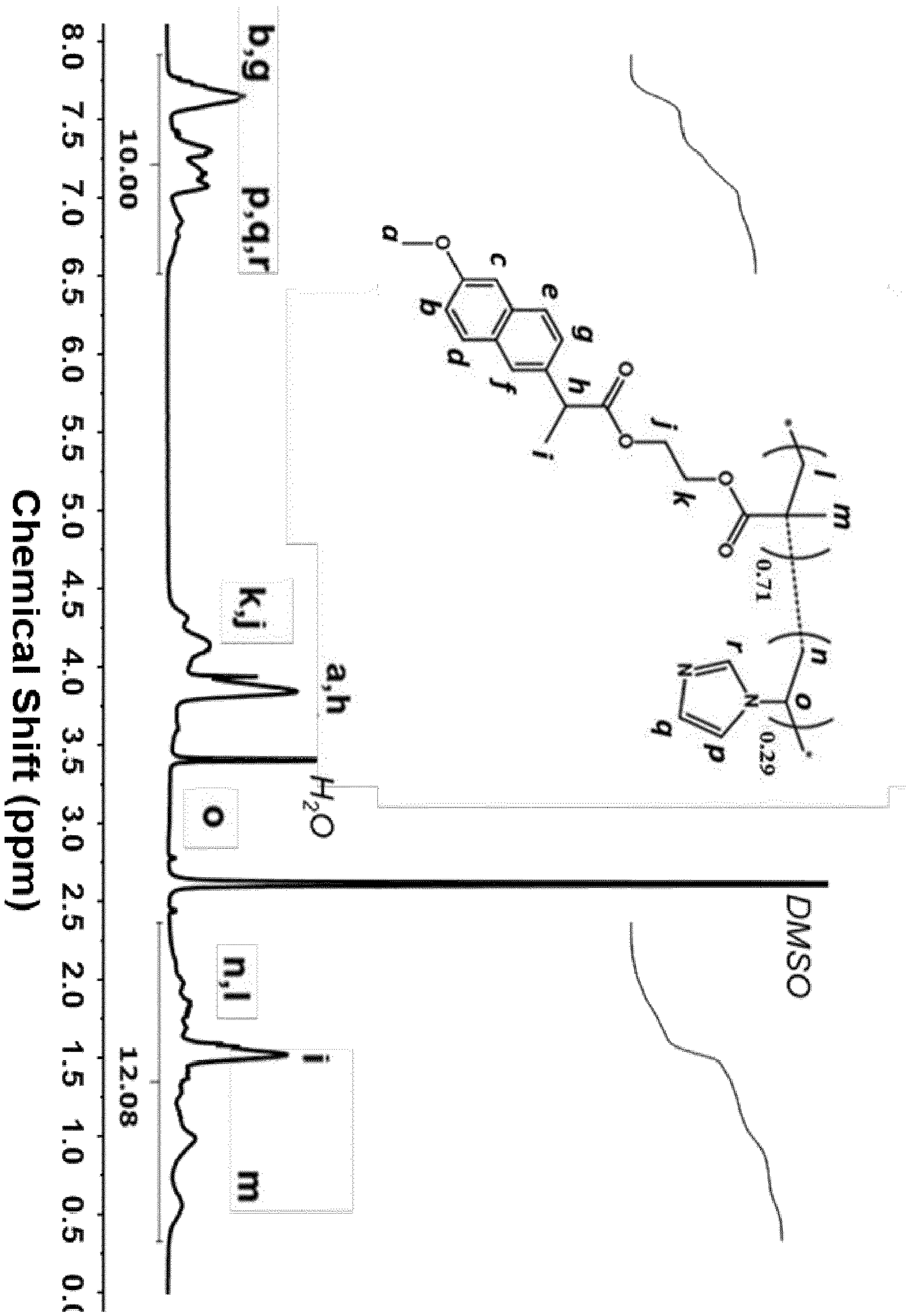

Chemical Shift (ppm)
8.0 7.5 7.0 6.5 6.0 5.5 5.0 4.5 4.0 3.5 3.0 2.5 2.0 1.5 1.0 0.5
10.00
12.08
DMSO
$H_2O$


FIG. 2

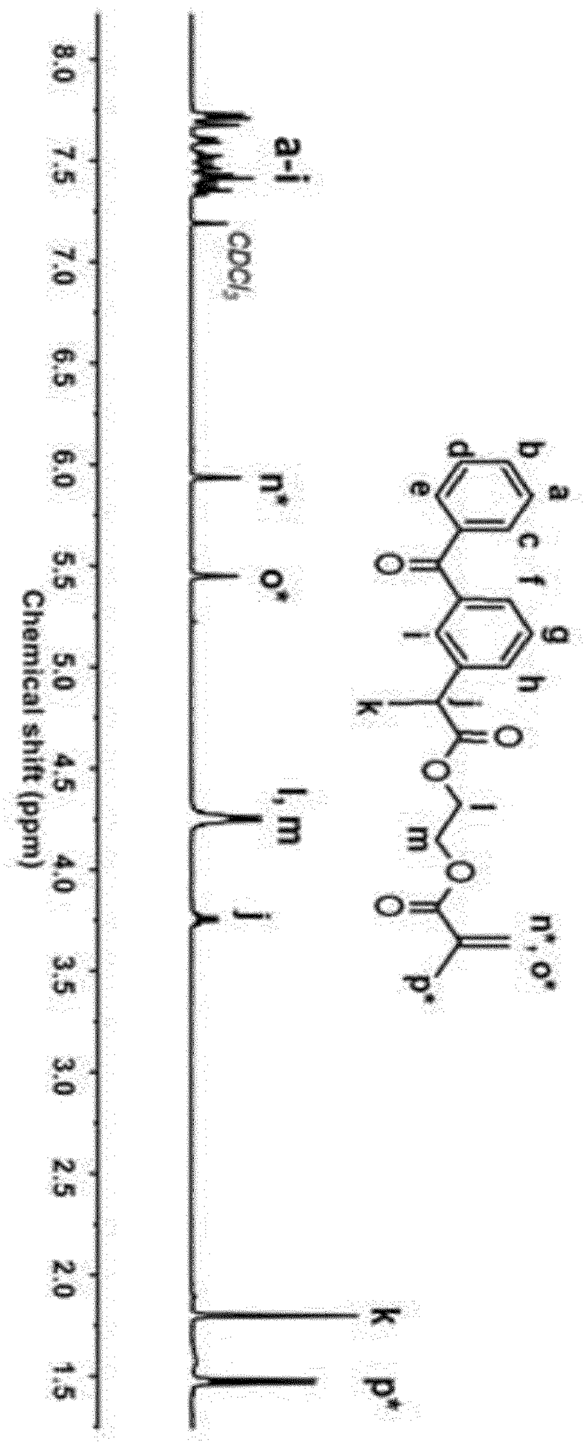


FIG. 3

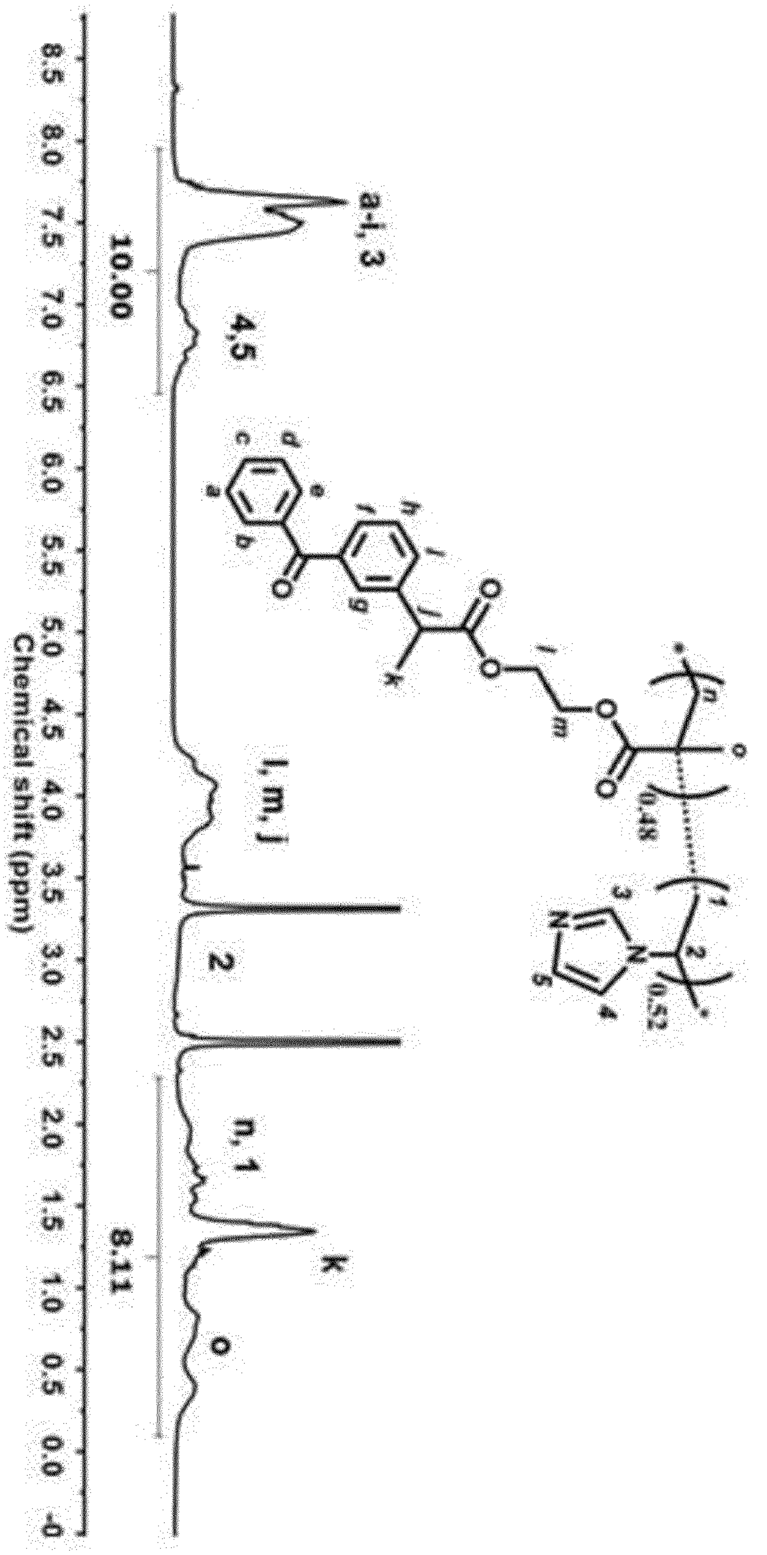


FIG. 4

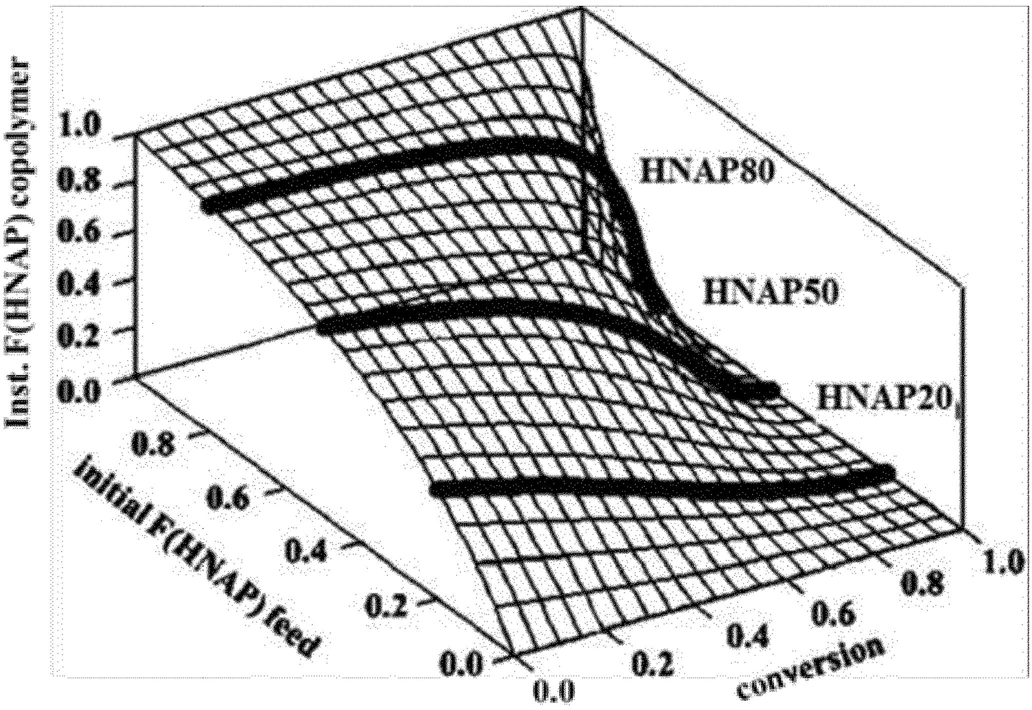
Inst. F(HNAP) copolymer
1.0
0.8
0.6
0.4
0.2
0.0
HNAP80
HNAP50
HNAP20
initial F(HNAP) feed
0.8
0.6
0.4
0.2
0.0
0.0
0.2
0.4
0.6
0.8
1.0
conversion

FIG. 5

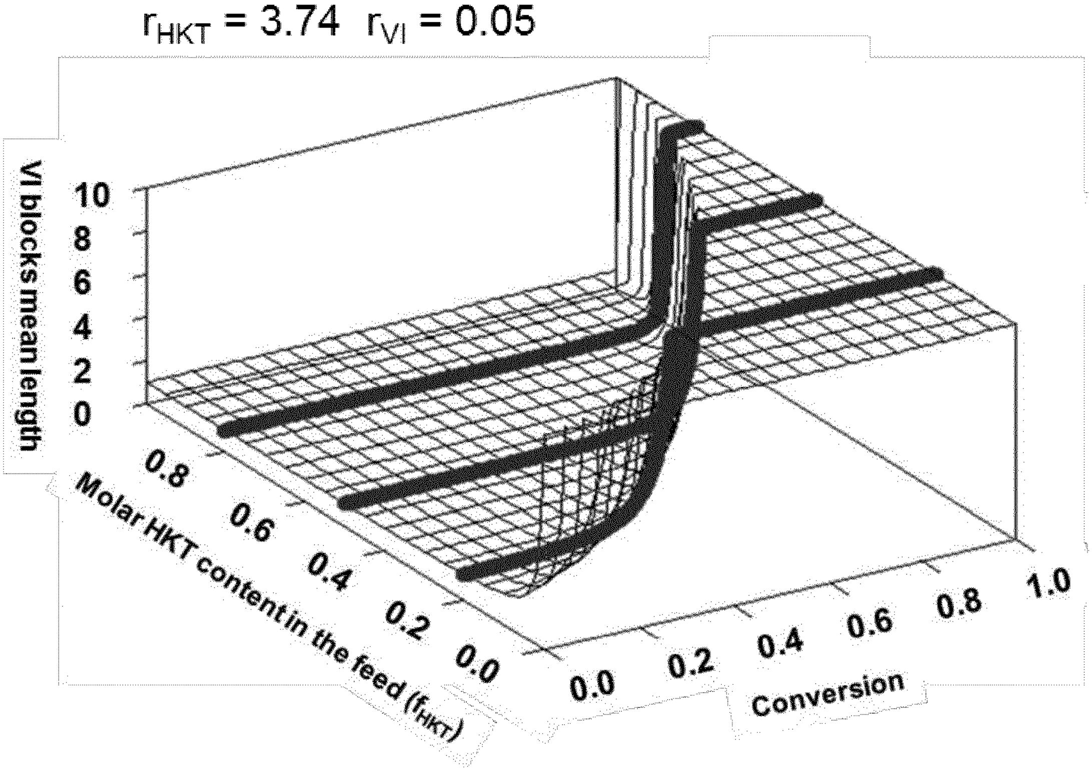

$r_{HKT}$ = 3.74 $r_{VI}$ = 0.05
VI blocks mean length
10
8
6
4
2
0
0.8
0.6
0.4
0.2
0.0
Molar HKT content in the feed ($f_{HKT}$)
0.0
0.2
0.4
0.6
0.8
1.0
Conversion


FIG. 6

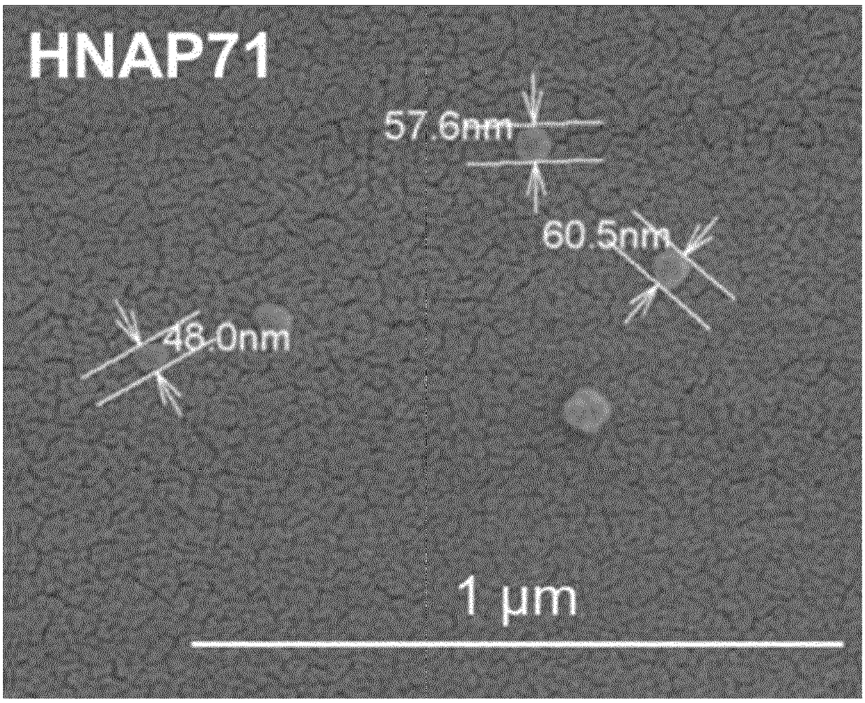

HNAP71
57.6nm
60.5nm
48.0nm
1 µm


FIG. 7

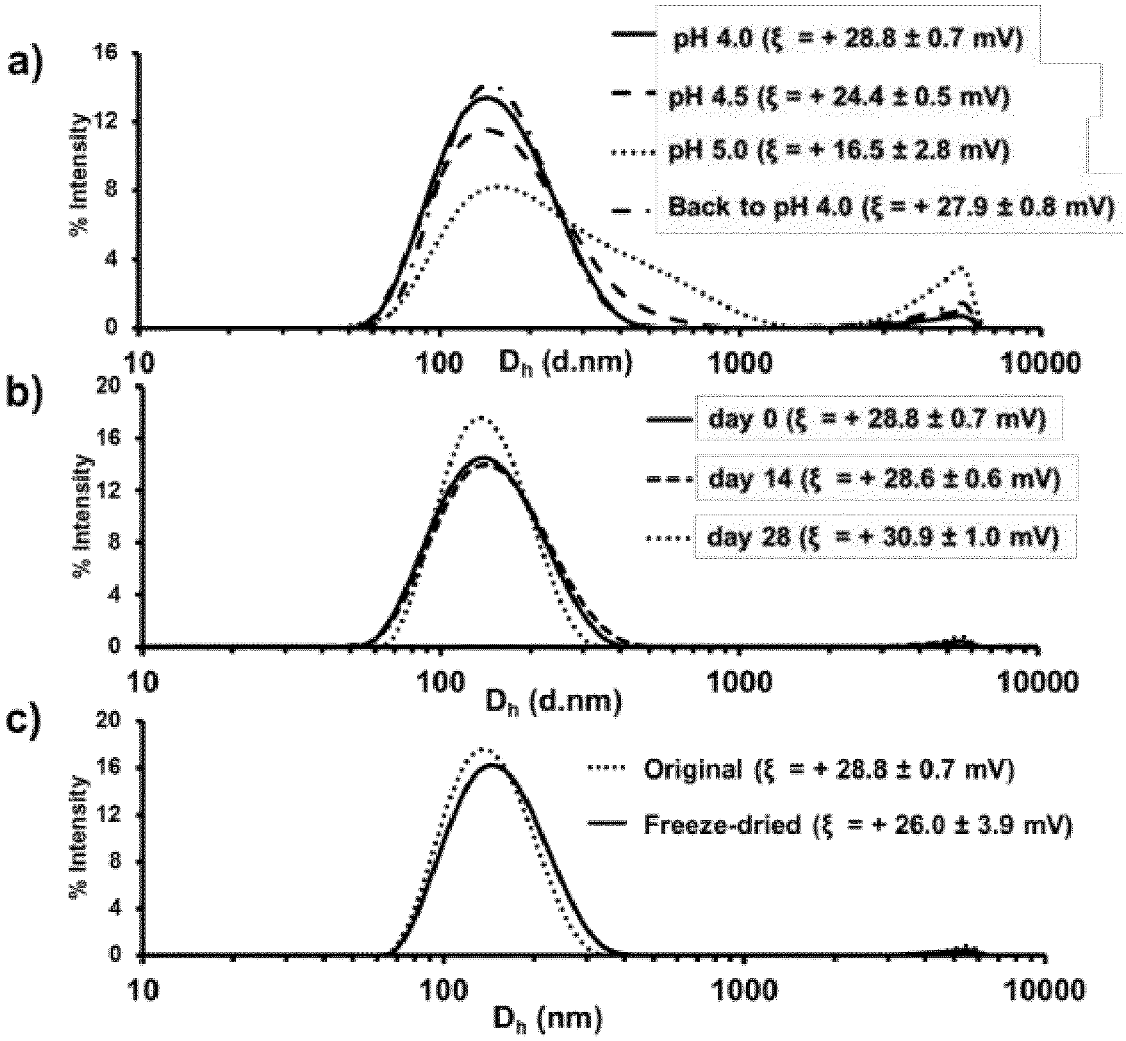
a)
16
12
8
4
0
% Intensity
pH 4.0 (ξ = + 28.8 ± 0.7 mV)
pH 4.5 (ξ = + 24.4 ± 0.5 mV)
pH 5.0 (ξ = + 16.5 ± 2.8 mV)
Back to pH 4.0 (ξ = + 27.9 ± 0.8 mV)
10
100
Dh (d.nm)
1000
10000
b)
20
16
12
8
4
0
% Intensity
day 0 (ξ = + 28.8 ± 0.7 mV)
day 14 (ξ = + 28.6 ± 0.6 mV)
day 28 (ξ = + 30.9 ± 1.0 mV)
10
100
Dh (d.nm)
1000
10000
c)
20
16
12
8
4
0
% Intensity
Original (ξ = + 28.8 ± 0.7 mV)
Freeze-dried (ξ = + 26.0 ± 3.9 mV)
10
100
Dh (nm)
1000
10000

FIG. 8

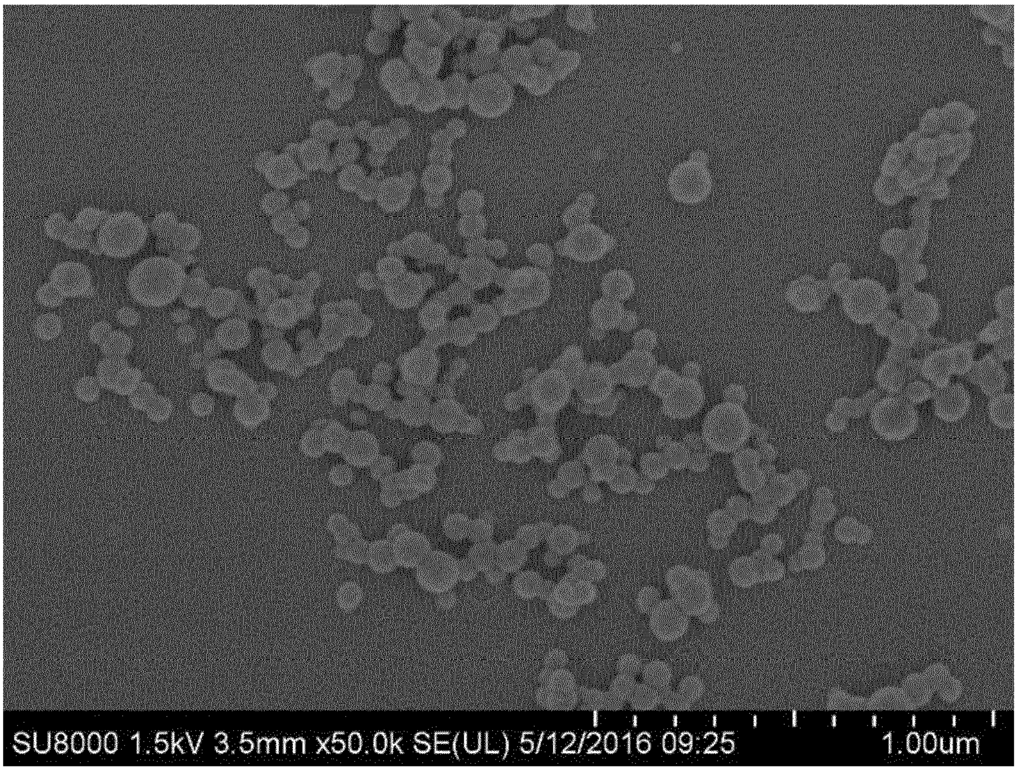


FIG. 9

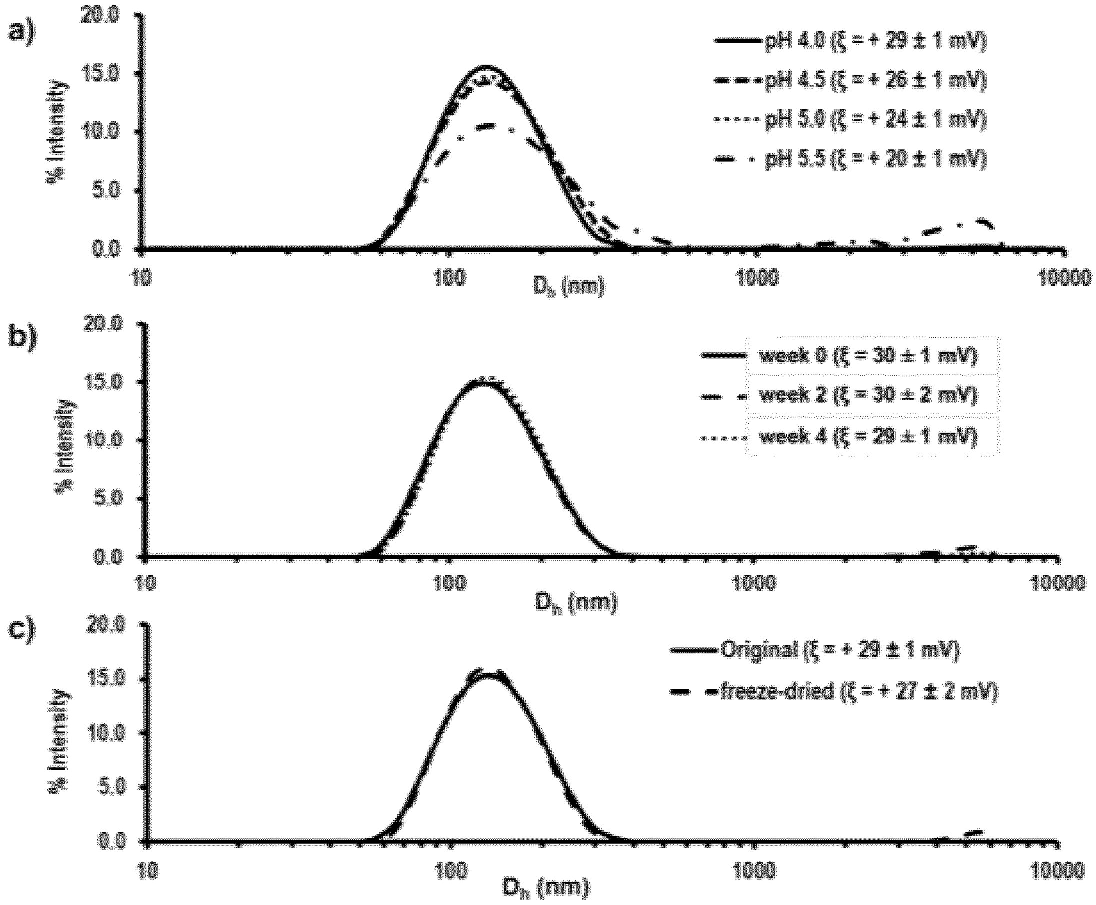
a)
20.0
15.0
10.0
5.0
0.0
% Intensity
10
100
1000
10000
D$_h$ (nm)
pH 4.0 (ξ = + 29 ± 1 mV)
pH 4.5 (ξ = + 26 ± 1 mV)
pH 5.0 (ξ = + 24 ± 1 mV)
pH 5.5 (ξ = + 20 ± 1 mV)
b)
week 0 (ξ = 30 ± 1 mV)
week 2 (ξ = 30 ± 2 mV)
week 4 (ξ = 29 ± 1 mV)
c)
Original (ξ = + 29 ± 1 mV)
freeze-dried (ξ = + 27 ± 2 mV)

FIG. 10

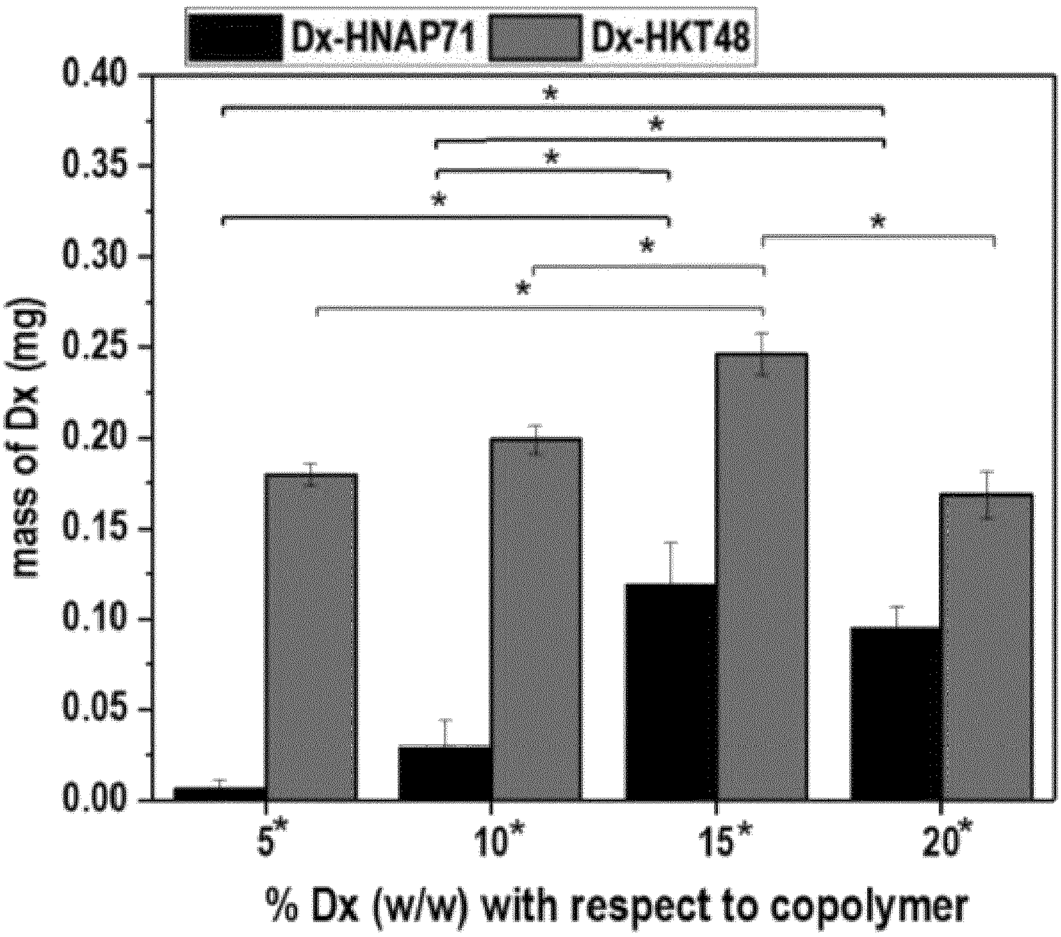
Dx-HNAP71
Dx-HKT48
mass of Dx (mg)
0.40
0.35
0.30
0.25
0.20
0.15
0.10
0.05
0.00
*
5*
10*
15*
20*
% Dx (w/w) with respect to copolymer

FIG. 11

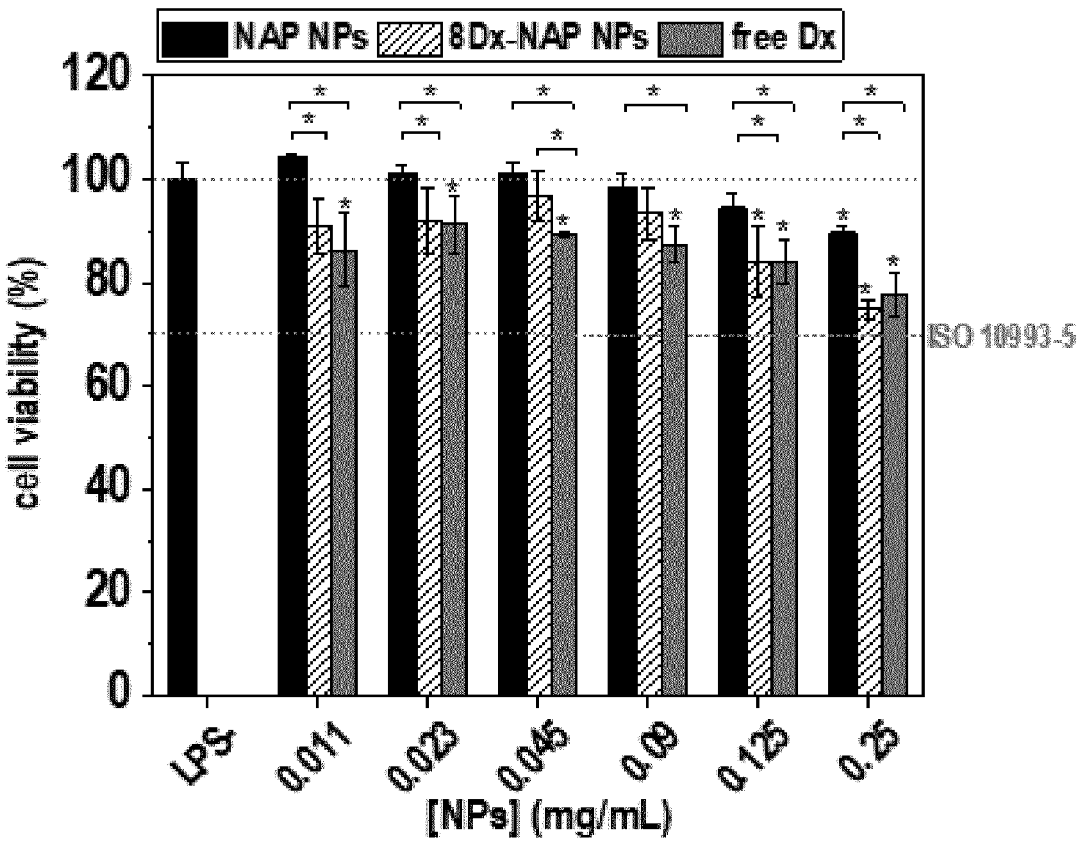
NAP NPs
8Dx-NAP NPs
free Dx
cell viability (%)
ISO 10993-5
LPS
0.011
0.023
0.045
0.09
0.125
0.25
[NPs] (mg/mL)

FIG. 12

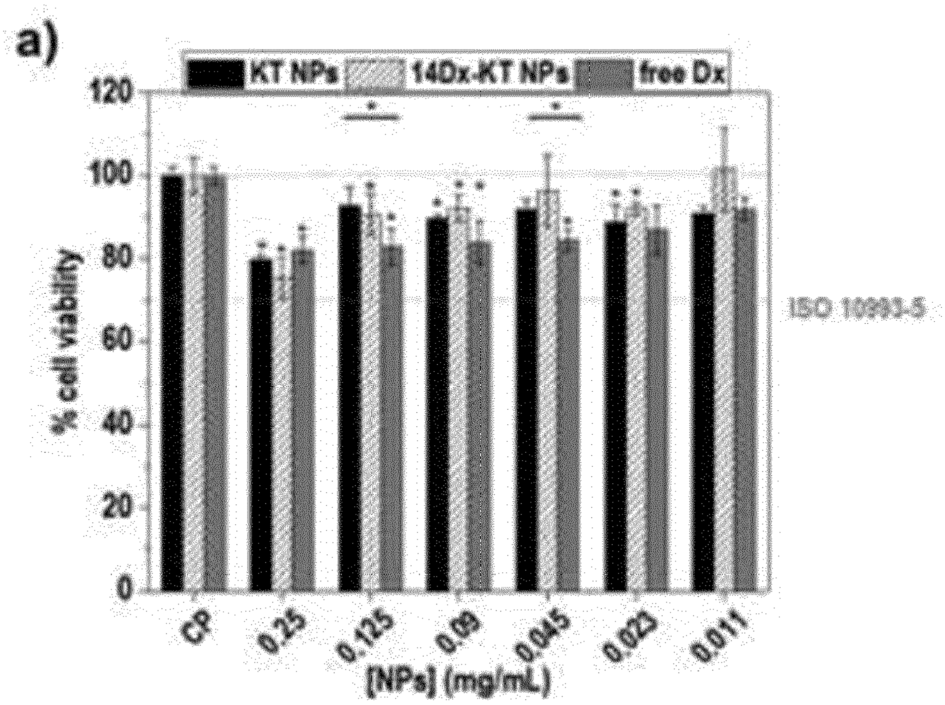
a)
KT NPs
14Dx-KT NPs
free Dx
% cell viability
ISO 10993-5
CP
0.25
0.125
0.09
0.045
0.023
0.011
[NPs] (mg/mL)

FIG. 13

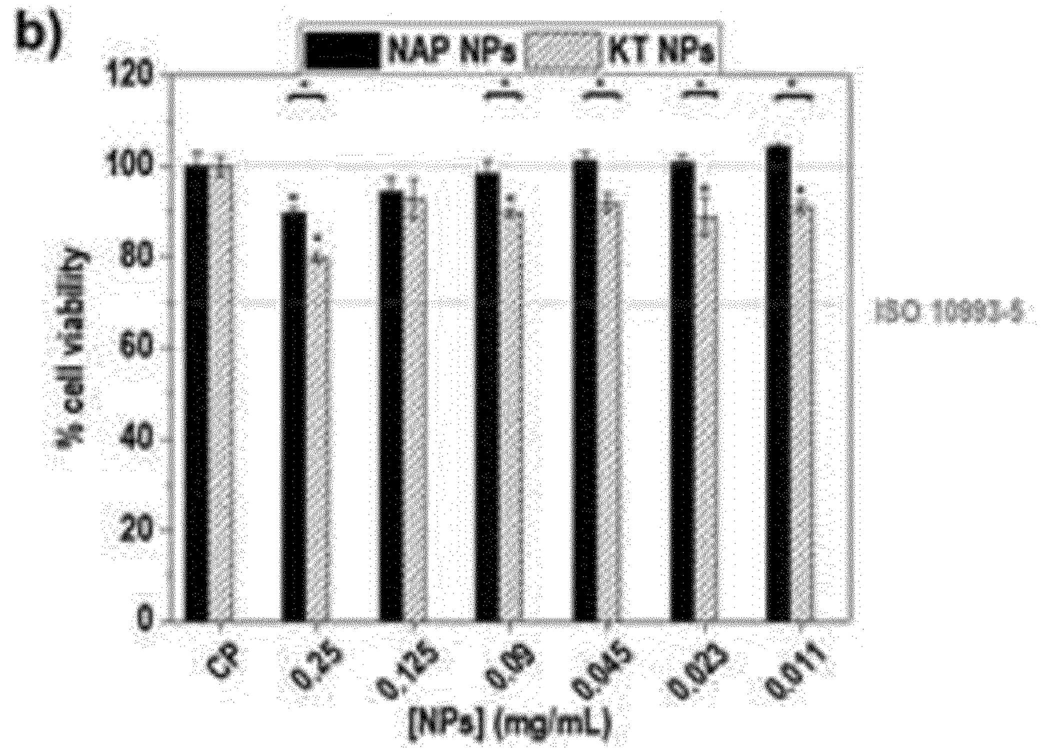
b)
NAP NPs
KT NPs
120
100
80
60
40
20
0
% cell viability
ISO 10993-5
CP
0.25
0.125
0.09
0.045
0.023
0.011
[NPs] (mg/mL)

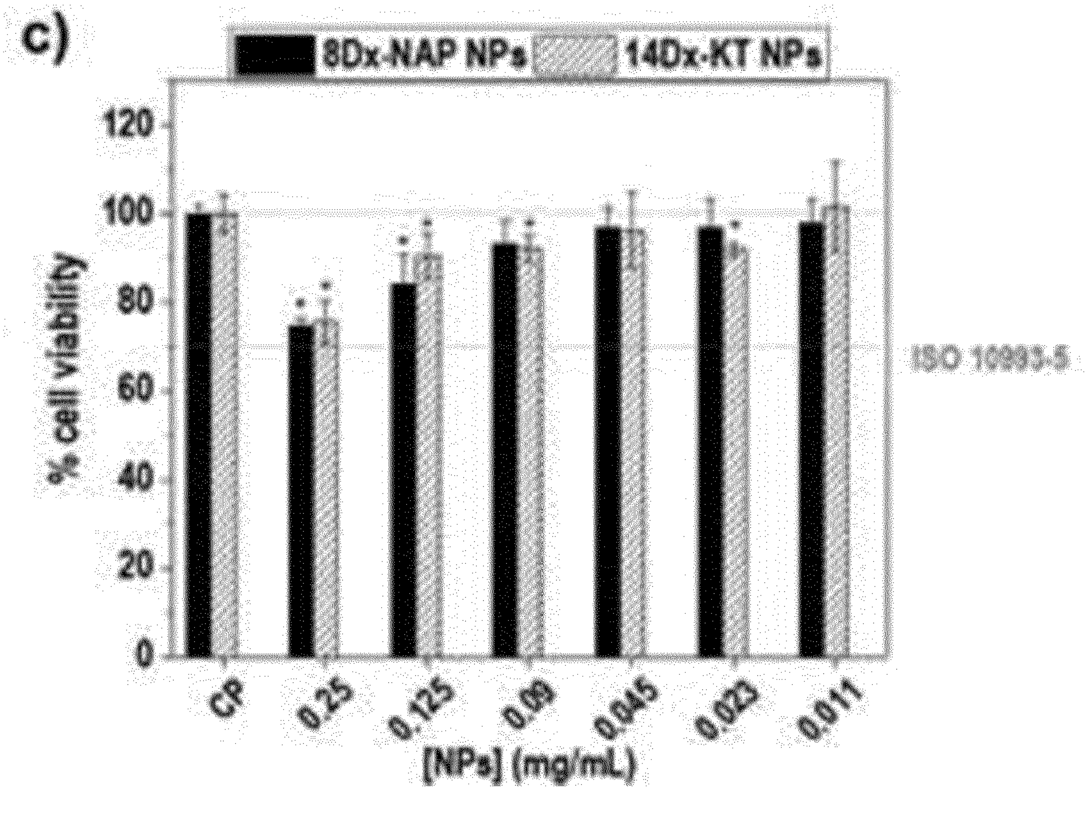
c)
8Dx-NAP NPs
14Dx-KT NPs
120
100
80
60
40
20
0
% cell viability
ISO 10993-5
CP
0.25
0.125
0.09
0.045
0.023
0.011
[NPs] (mg/mL)

FIG. 13 (Cont.)

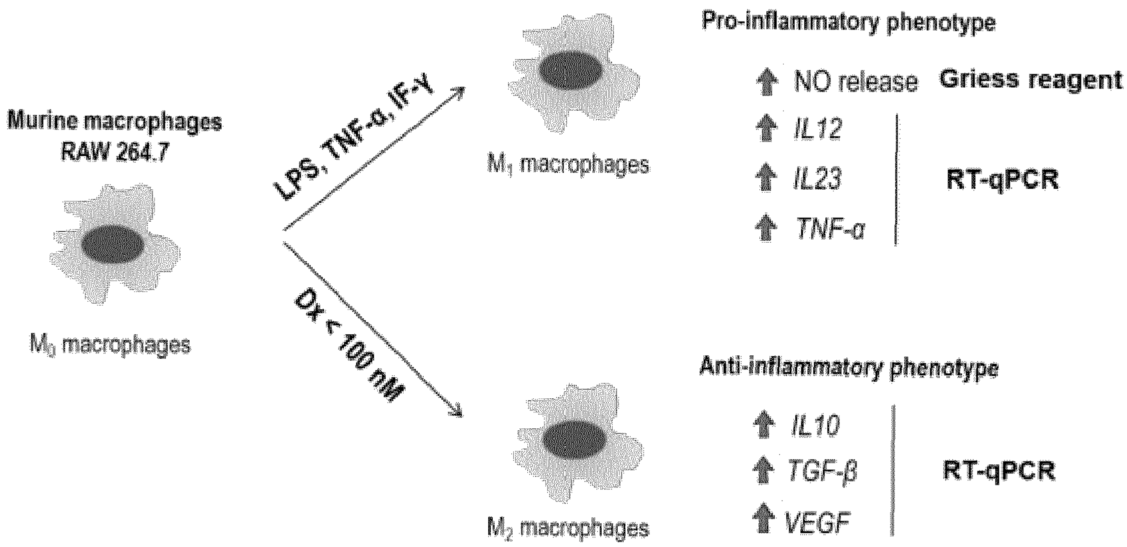
Murine macrophages
RAW 264.7
M0 macrophages
LPS, TNF-α, IF-γ
Dx < 100 nM
M1 macrophages
M2 macrophages
Pro-inflammatory phenotype
NO release
Griess reagent
IL12
IL23
TNF-α
RT-qPCR
Anti-inflammatory phenotype
IL10
TGF-β
VEGF
RT-qPCR

FIG. 14

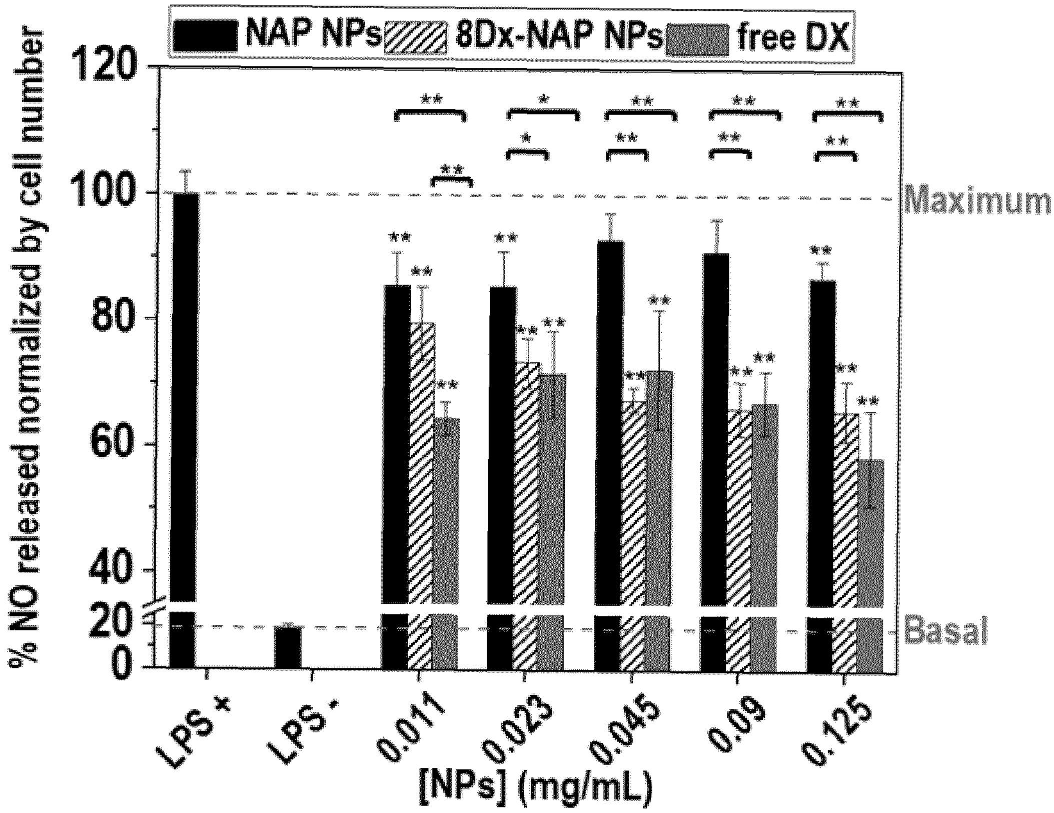
NAP NPs
8Dx-NAP NPs
free DX
% NO released normalized by cell number
Maximum
Basal
LPS +
LPS -
0.011
0.023
0.045
0.09
0.125
[NPs] (mg/mL)

FIG. 15

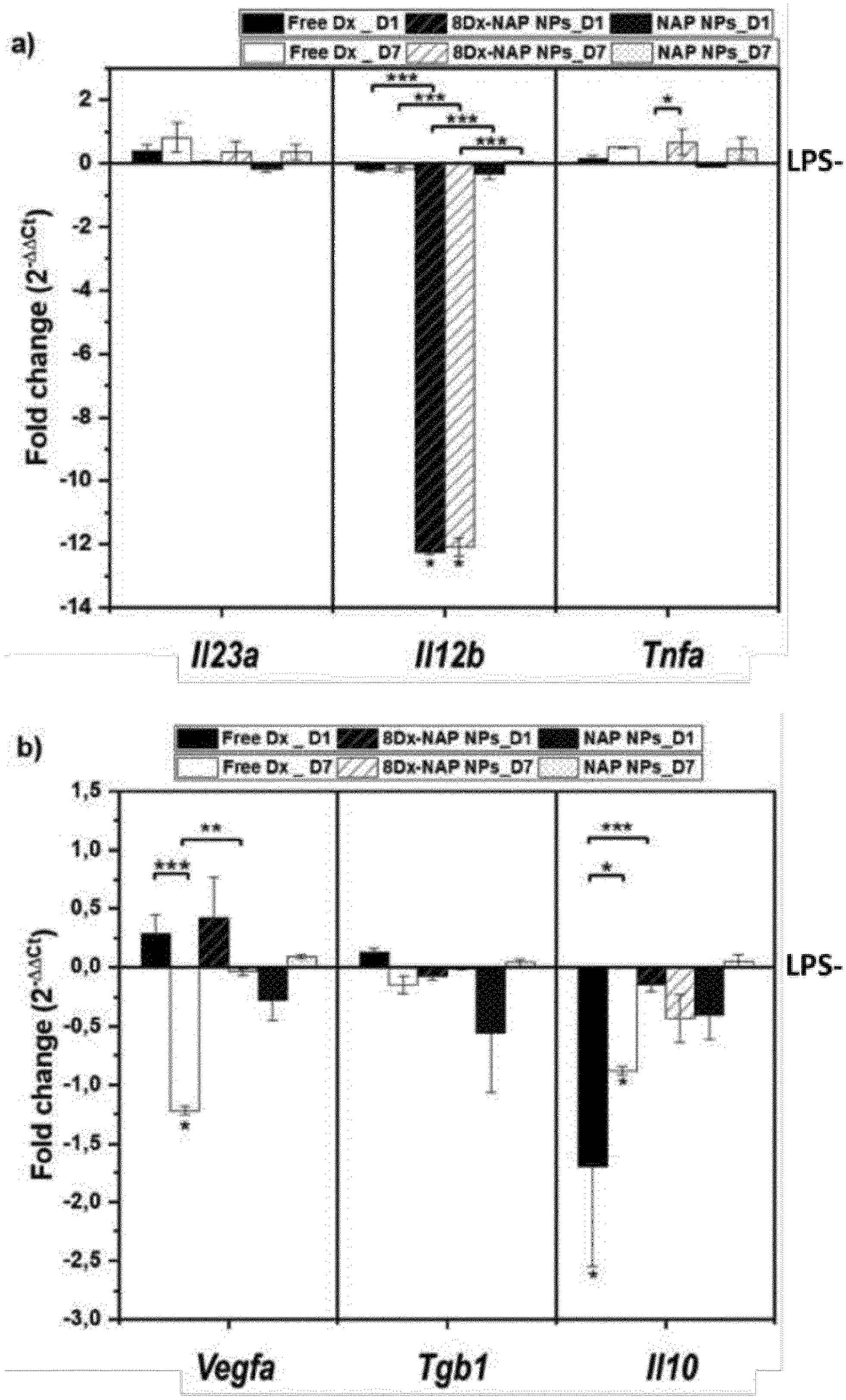
a)
Free Dx _ D1
8Dx-NAP NPs_D1
NAP NPs_D1
Free Dx _ D7
8Dx-NAP NPs_D7
NAP NPs_D7
Fold change ($2^{-\Delta\Delta Ct}$)
2
0
-2
-4
-6
-8
-10
-12
-14
***
*
LPS-
Il23a
Il12b
Tnfa
b)
Free Dx _ D1
8Dx-NAP NPs_D1
NAP NPs_D1
Free Dx _ D7
8Dx-NAP NPs_D7
NAP NPs_D7
Fold change ($2^{-\Delta\Delta Ct}$)
1,5
1,0
0,5
0,0
-0,5
-1,0
-1,5
-2,0
-2,5
-3,0
***
**
*
LPS-
Vegfa
Tgb1
Il10

FIG. 16

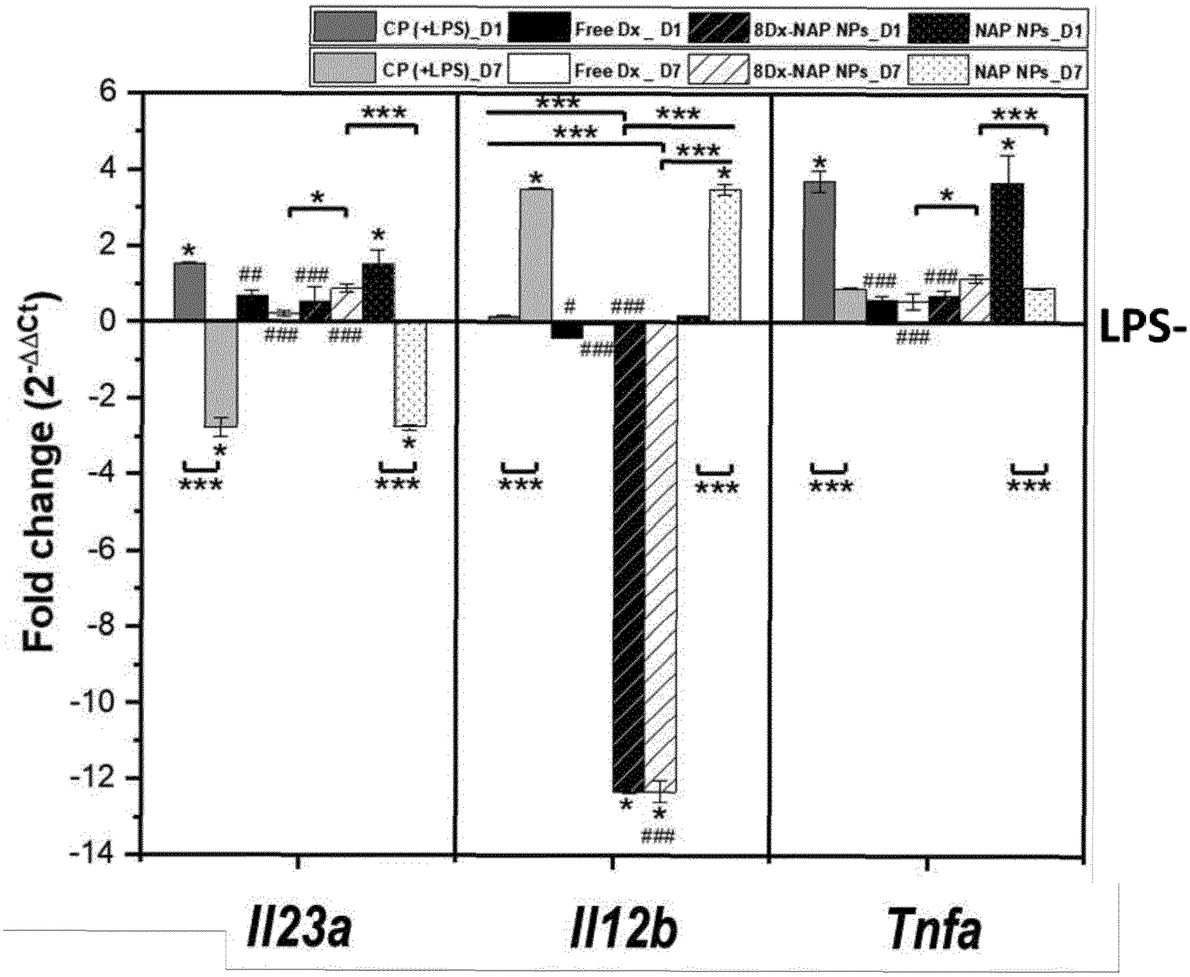
CP (+LPS)_D1
Free Dx _ D1
8Dx-NAP NPs_D1
NAP NPs_D1
CP (+LPS)_D7
Free Dx _ D7
8Dx-NAP NPs_D7
NAP NPs_D7
Fold change ($2^{-\Delta\Delta Ct}$)
LPS-
Il23a
Il12b
Tnfa

FIG. 17

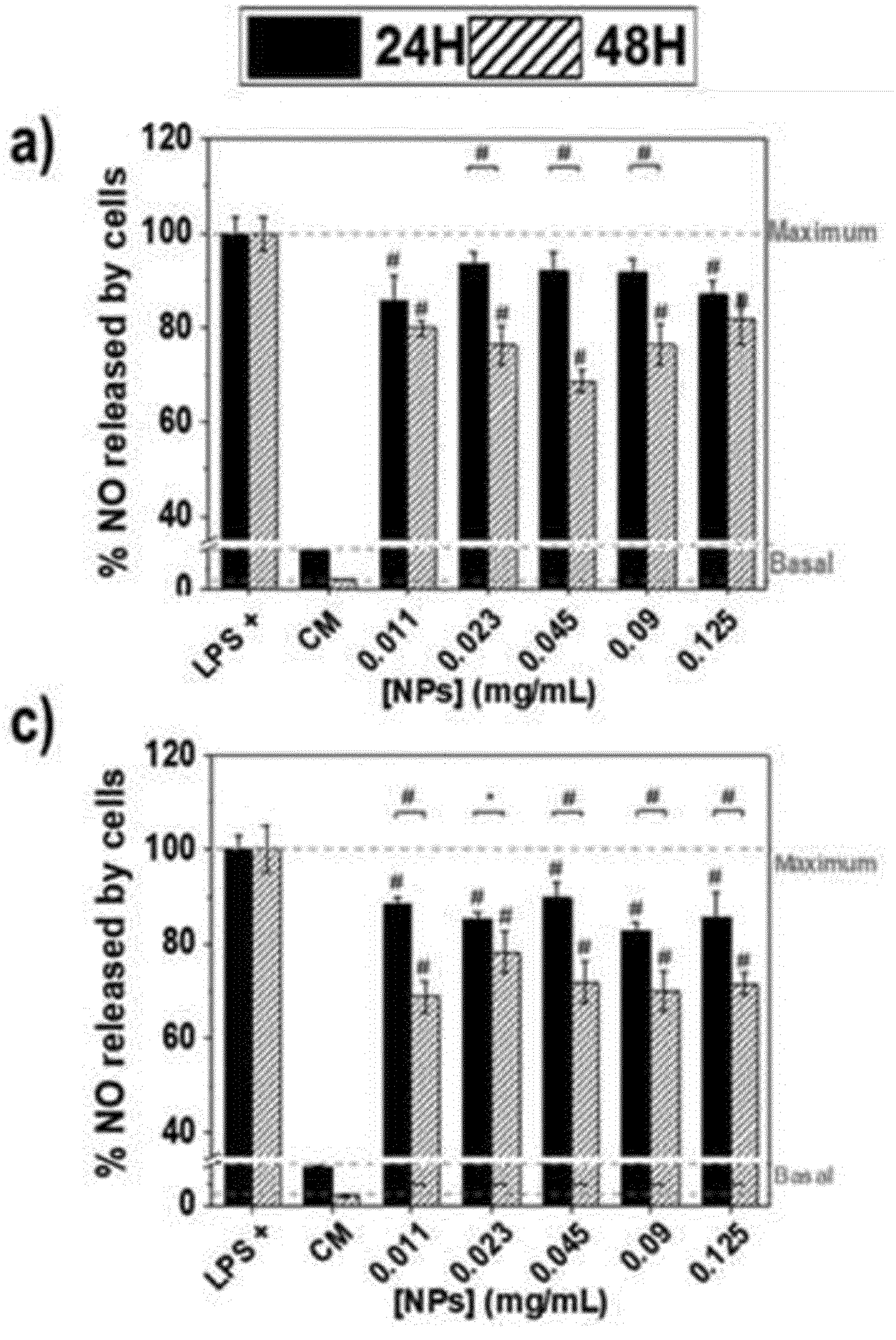
24H
48H
a)
% NO released by cells
120
100
80
60
40
0
Maximum
Basal
LPS +
CM
0.011
0.023
0.045
0.09
0.125
[NPs] (mg/mL)
c)
% NO released by cells
120
100
80
60
40
0
Maximum
Basal
LPS +
CM
0.011
0.023
0.045
0.09
0.125
[NPs] (mg/mL)

FIG. 18

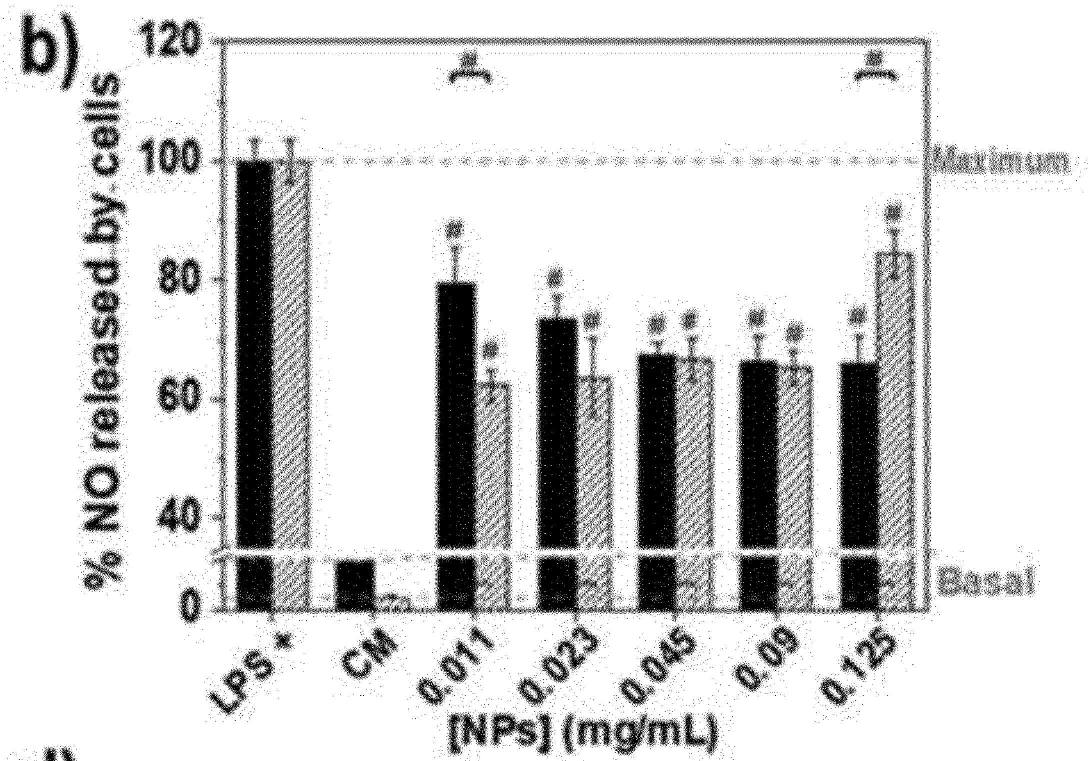
b)
% NO released by cells
120
100
80
60
40
0
Maximum
Basal
#
LPS +
CM
0.011
0.023
0.045
0.09
0.125
[NPs] (mg/mL)

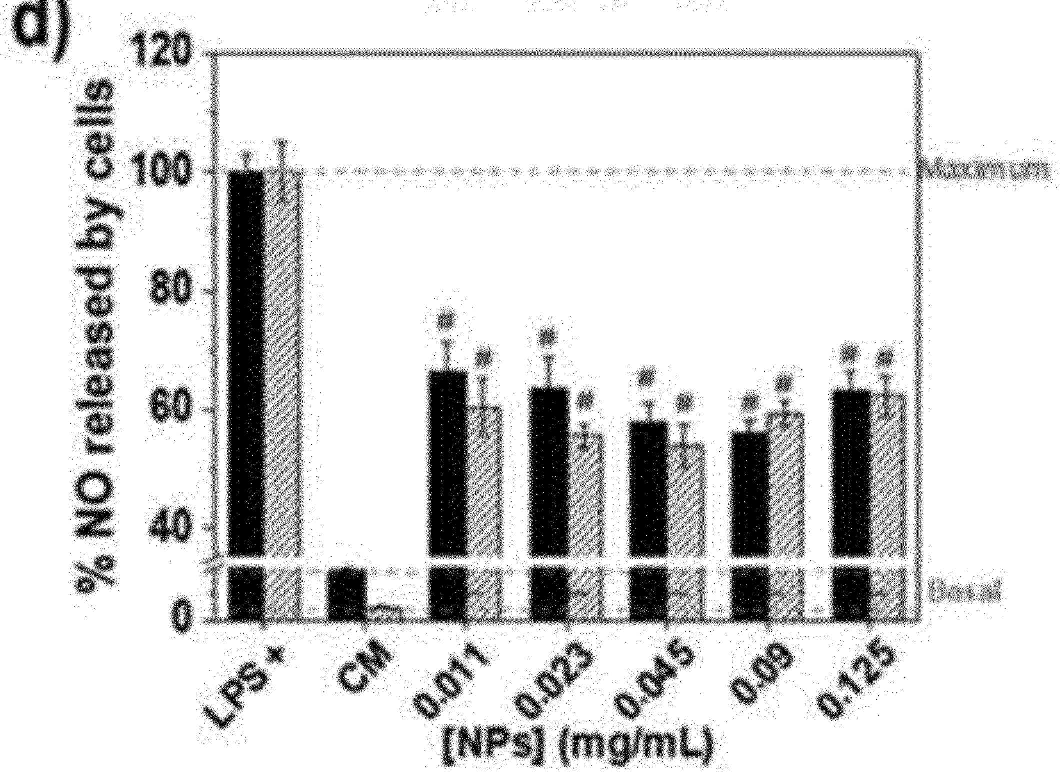
d)
% NO released by cells
120
100
80
60
40
0
Maximum
Basal
#
LPS +
CM
0.011
0.023
0.045
0.09
0.125
[NPs] (mg/mL)

FIG. 18 (Cont.)

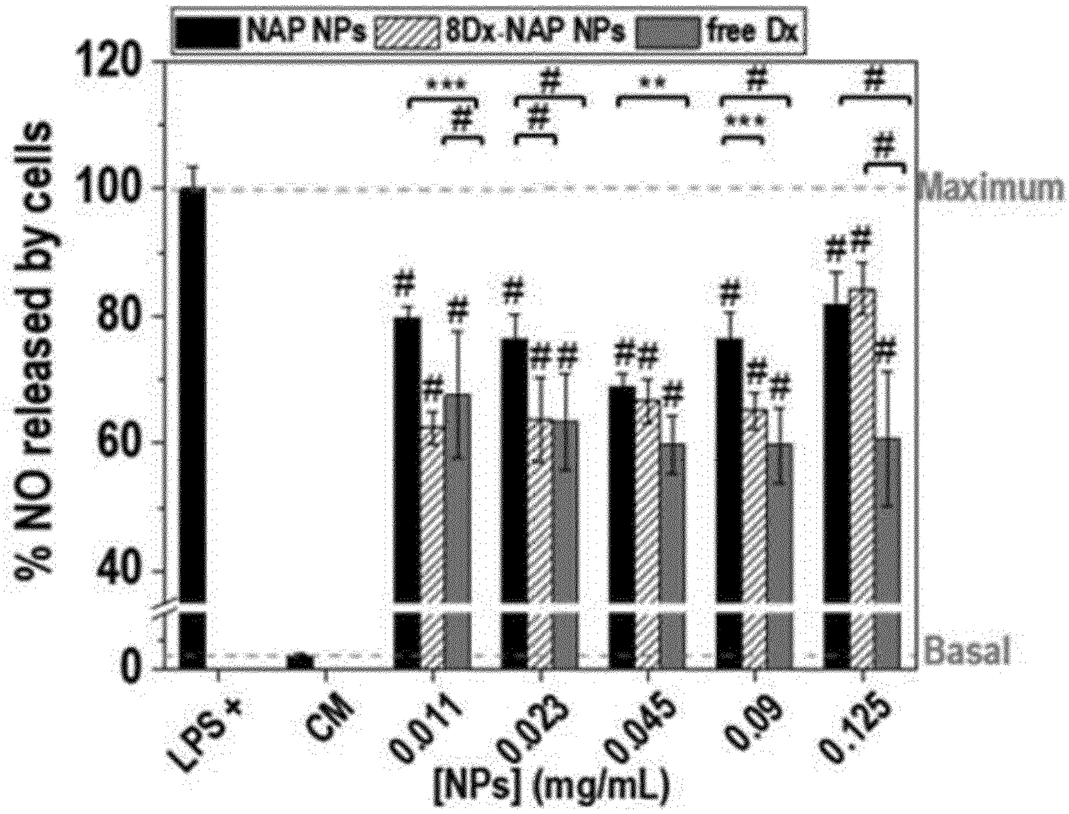
NAP NPs
8Dx-NAP NPs
free Dx
% NO released by cells
120
100
80
60
40
0
***
#
**
Maximum
Basal
LPS +
CM
0.011
0.023
0.045
0.09
0.125
[NPs] (mg/mL)

FIG. 19

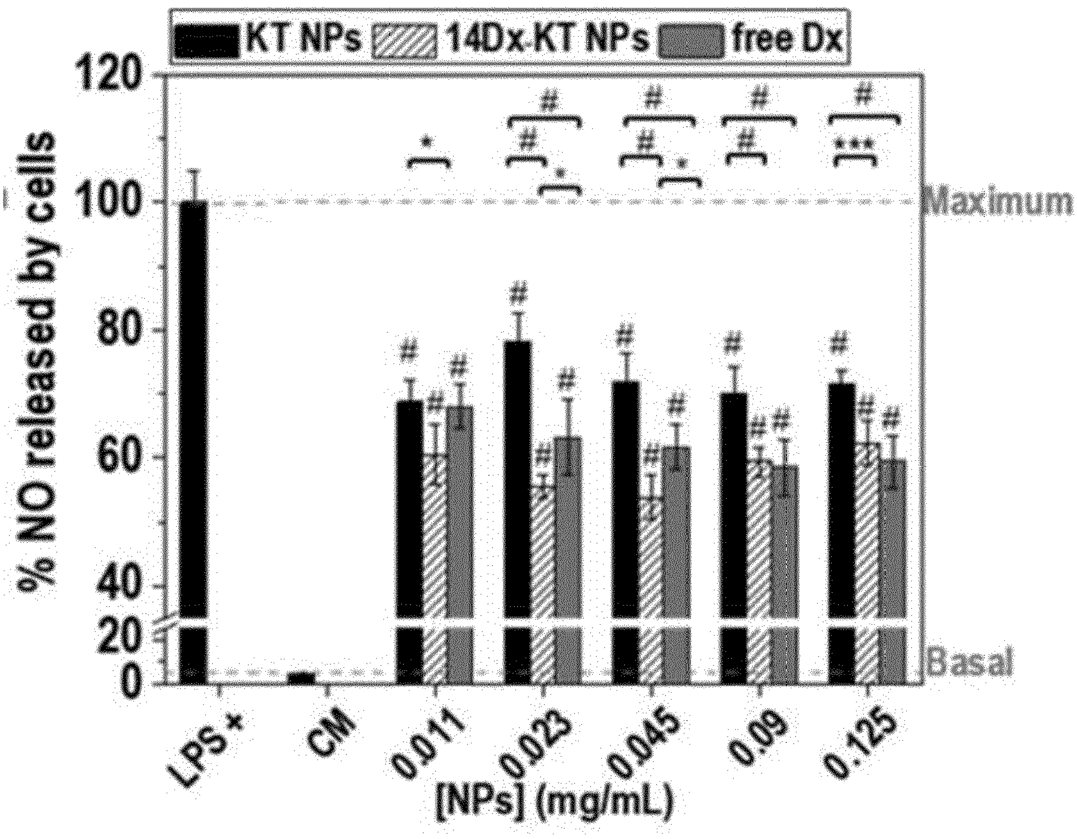
KT NPs
14Dx-KT NPs
free Dx
% NO released by cells
120
100
80
60
40
20
0
Maximum
Basal
LPS +
CM
0.011
0.023
0.045
0.09
0.125
[NPs] (mg/mL)

FIG. 19 (Cont.)

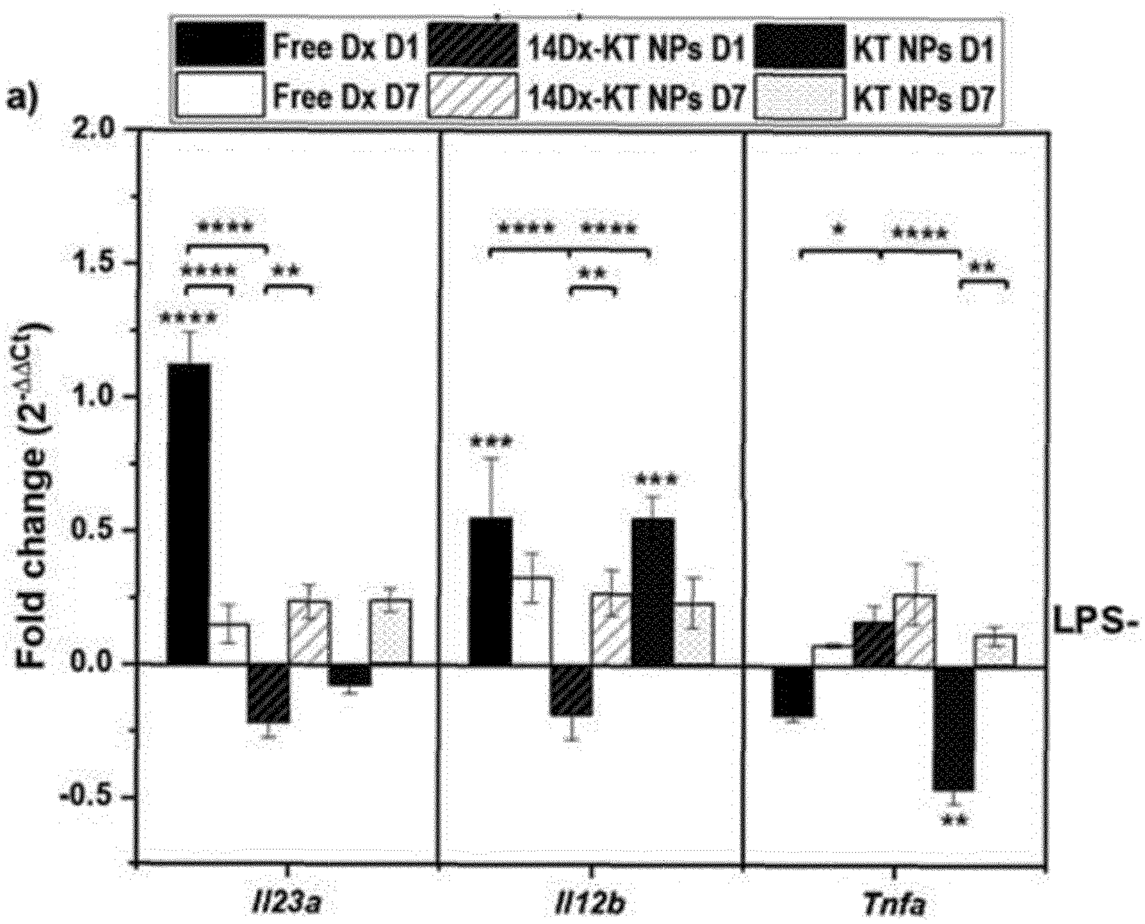
a)
Free Dx D1
Free Dx D7
14Dx-KT NPs D1
14Dx-KT NPs D7
KT NPs D1
KT NPs D7
Fold change ($2^{-\Delta\Delta Ct}$)
2.0
1.5
1.0
0.5
0.0
-0.5
****
**
***
*
LPS-
Il23a
Il12b
Tnfa

FIG. 20

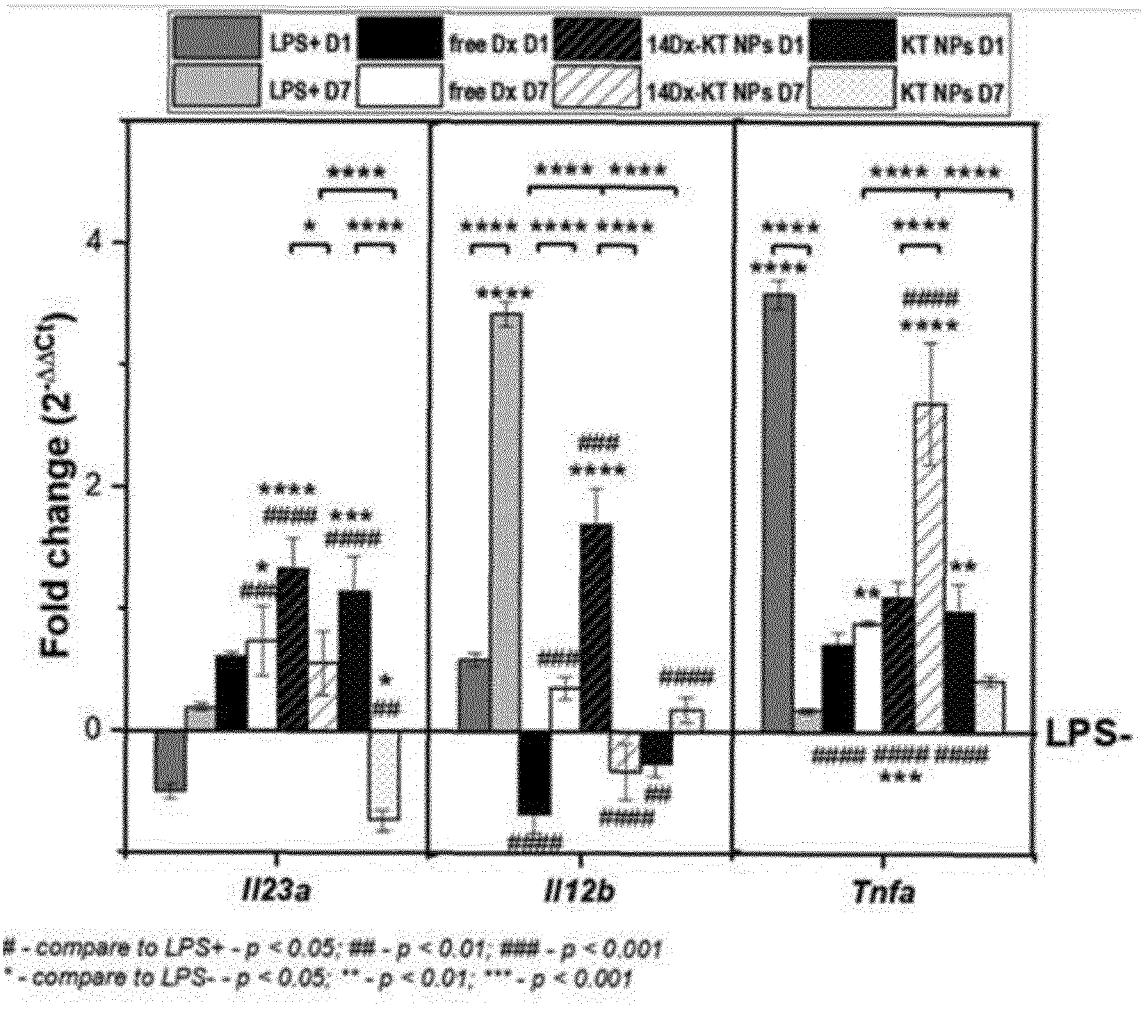
LPS+ D1
free Dx D1
14Dx-KT NPs D1
KT NPs D1
LPS+ D7
free Dx D7
14Dx-KT NPs D7
KT NPs D7
Fold change ($2^{-\Delta\Delta Ct}$)
LPS-
Il23a
Il12b
Tnfa
# - compare to LPS+ - p < 0.05; ## - p < 0.01; ### - p < 0.001
* - compare to LPS- - p < 0.05; ** - p < 0.01; *** - p < 0.001

FIG. 20 (Cont.)

Europäisches Patentamt
European Patent Office
Office européen des brevets

# EUROPEAN SEARCH REPORT

Application Number
EP 20 38 2234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Diego Velasco ET AL: "Actas del XLI Congreso de la Sociedad Ibérica de Biomecánica y Biomateriales Madrid, 18-19 de Octubre de 2018", , 1 April 2019 (2019-04-01), XP055728677, Retrieved from the Internet: URL:https://www.researchgate.net/publication/332298644_Actas_del_XLI_Congreso_de_la_Sociedad_Iberica_de_Biomecanica_y_Biomateriales_Madrid_18-19_de_Octubre_de_2018 [retrieved on 2020-09-08] * page 57 * ----- | 1-15 | INV. A61K47/69 A61P29/00 A61K9/51 |
| A | Juan Garcia-Lopez ET AL: "Actas del XXXIX Congreso de la Sociedad Ibérica de Biomecánica y Biomateriales, SIBB 2016", , 1 October 2016 (2016-10-01), XP055728678, Retrieved from the Internet: URL:https://www.researchgate.net/publication/315670450_Actas_del_XXXIX_Congreso_de_la_Sociedad_Iberica_de_Biomecanica_y_Biomateriales_SIBB_2016 [retrieved on 2020-09-08] * page 68 * ----- | 1-15 | TECHNICAL FIELDS SEARCHED (IPC): A61K A61P |
| | -/-- | | |

The present search report has been drawn up for all claims

| Place of search | Date of completion of the search | Examiner |
|---|---|---|
| The Hague | 9 September 2020 | Burema, Shiri |

1

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 3

Europäisches Patentamt
European Patent Office
Office européen des brevets

# EUROPEAN SEARCH REPORT

Application Number
EP 20 38 2234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HALA AL-KHOURY ET AL: "Anti-inflammatory Surface Coatings Based on Polyelectrolyte Multilayers of Heparin and Polycationic Nanoparticles of Naproxen-Bearing Polymeric Drugs", BIOMACROMOLECULES, vol. 20, no. 10, 26 August 2019 (2019-08-26), pages 4015-4025, XP055728690, ISSN: 1525-7797, DOI: 10.1021/acs.biomac.9b01098 * the whole document * ----- | 1-15 | |
| A | ES 2 391 187 A1 (CONSEJO SUPERIOR INVESTIGACION [ES]) 22 November 2012 (2012-11-22) * the whole document * ----- | 1-15 | |
| A | EP 3 583 939 A1 (ALODIA FARM S L [ES] ET AL.) 25 December 2019 (2019-12-25) * the whole document * ----- | 1-15 | TECHNICAL FIELDS SEARCHED (IPC) |
| A | WO 2013/167782 A1 (CONSEJO SUPERIOR INVESTIGACION [ES] ET AL.) 14 November 2013 (2013-11-14) * the whole document * ----- | 1-15 | |
| A | SERGIO MARTÍN-SALDAÑA ET AL: "Polymeric nanoparticles loaded with dexamethasone or [alpha]-tocopheryl succinate to prevent cisplatin-induced ototoxicity", ACTA BIOMATERIALIA, vol. 53, 14 February 2017 (2017-02-14), pages 199-210, XP055729202, AMSTERDAM, NL ISSN: 1742-7061, DOI: 10.1016/j.actbio.2017.02.019 * the whole document * ----- -/-- | 1-15 | |

The present search report has been drawn up for all claims

1

| Place of search | Date of completion of the search | Examiner |
|---|---|---|
| The Hague | 9 September 2020 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 20 38 2234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | - Anonymous: "Memoria 2018 - Instituto de Ciencia y Tecnologia de Polimeros", , 31 December 2018 (2018-12-31), XP055728704, Retrieved from the Internet: URL:http://www.ictp.csic.es/ICTP2/sites/default/files/MEMORIA2018_0.pdf [retrieved on 2020-09-08] * pages 59-65 * ----- | 1-15 | |
| | | | TECHNICAL FIELDS SEARCHED (IPC) |

1

The present search report has been drawn up for all claims

| Place of search | Date of completion of the search | Examiner |
|---|---|---|
| The Hague | 9 September 2020 | Burema, Shiri |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT ON EUROPEAN PATENT APPLICATION NO. EP 20 38 2234

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2020

| Patent document cited in search report | Publication date | Patent family member(s) | Publication date |
|---|---|---|---|
| ES 2391187 A1 | 22-11-2012 | NONE | |
| EP 3583939 A1 | 25-12-2019 | EP 3583939 A1 | 25-12-2019 |
| | | ES 2735638 A1 | 19-12-2019 |
| WO 2013167782 A1 | 14-11-2013 | ES 2433248 A1 | 10-12-2013 |
| | | WO 2013167782 A1 | 14-11-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHENG, C. J. ; TIETJEN, G. T. ; SAUCIER-SAWYER, J. K. ; SALTZMAN, W. M.** *Nature reviews. Drug discovery,* 2015, vol. 14 (4), 239-247
- **AWAAD, A. ; NAKAMURA, M. ; ISHIMURA, K.** *Nanomedicine,* 2012, vol. 8 (5), 627-636
- **SUÁREZ, P. et al.** *Macromol. Biosci.,* 2013, vol. 13 (9
- *Macromol. Biosci.,* 2013, vol. 13 (9
- **AGUILAR, M. R. ; GALLARDO, A. ; FERNÁNDEZ, M. D. M. ; ROMAN, J. S.** *Macromolecules,* 2002, vol. 35 (6), 2036-2041